# EUROPEAN PATENT APPLICATION

(11) **EP 1 306 387 A1**
(43) Date of publication of application: **02.05.2003**
(21) Application number: 01947980.7
(22) Date of filing: 12.07.2001
(51) Int. Cl.: C08B 37/00, A61K 31/737, A61K 35/56, A61K 35/80, A61K 7/00, A61P 17/00

(54) **DRUGS OR COSMETICS**

(30) Priority: 13.07.2000 JP 2000212143; 28.12.2000 JP 2000400615; 09.03.2001 JP 2001067445
(71) Applicant: TAKARA BIO INC., Otsu-shi, Shiga 520-2193 (JP)
(72) Inventor: WU, Hua-Kang, Otsu-shi, Shiga 520-2133 (JP); SAKAI, Takeshi, Hirosaki-shi, Aomori 036-8183 (JP); ADACHI, Shinichi, Hirakata-shi, Osaka 573-1178 (JP); YASUDA, Mariko, Kyoto-shi, Kyoto 612-8073 (JP); KATO, Ikunoshin, Uji-shi, Kyoto 611-0028 (JP)
(74) Representative: VOSSIUS & PARTNER
(86) International application number: JP0106032
(87) International publication number: WO02006351

(57) **Abstract**

The present invention provides a medicament for use as any one of a therapeutic agent or prophylactic agent for a disease requiring enhancement of transforming growth factor-β production, an agent for amelioration or prevention of wrinkle, an agent for improvement or sustenance of skin elasticity, an agent for amelioration or prevention of epidermal thickening, and a suppressor for decreasing collagen or enhancer for collagen production, and cosmetics used for any one of enhancement of transforming growth factor-β production, amelioration or prevention of wrinkle, improvement or sustenance of skin elasticity, amelioration or prevention of epidermal thickening, and suppression for decreasing collagen or enhancement for collagen production, and the like, characterized in that each comprises as an effective ingredient one or more members selected from the group consisting of a fucoidan, a degradation product thereof and a salt thereof.

## Description

### TECHNICAL FIELD

The present invention especially relates to applications of physiologically active substances derived from aquatic organisms as medicaments or cosmetics.

### BACKGROUND ART

As physiologically active substances derived from aquatic organisms, a fucoidan has been known. This fucoidan is a sulfated fucose-containing polysaccharide contained in algae, Echinodermata, or the like. The fucoidan comprises a sulfated fucose as a constituting saccharide.

As physiological action of the fucoidan, cancer proliferation-suppressing activity, cancer metastasis-suppressing activity, anticoagulation activity, antiviral activity and the like have been known, and the developments of applications as medicaments or the like have been expected for the fucoidan.

The present invention is concerned with new physiological action of the fucoidan, and its object is to provide a medicament or cosmetics, utilizing the fucoidan, a degradation product thereof or a salt thereof.

### DISCLOSURE OF INVENTION

Summarizing the present invention, a first invention of the present invention relates to a medicament for use as any one of a therapeutic agent or prophylactic agent for a disease requiring enhancement of transforming growth factor-β production, an agent for amelioration or prevention of wrinkle, an agent for improvement or sustenance of skin elasticity, an agent for amelioration or prevention of epidermal thickening, and a suppressor for decreasing collagen or enhancer for collagen production, characterized in that the medicament comprises as an effective ingredient one or more members selected from the group consisting of a fucoidan, a degradation product thereof and a salt thereof. In the present specification, the subject matter of the first invention of the present invention may be simply referred to as "medicament" in some cases.

A second invention of the present invention relates to cosmetics used for any one of enhancement of transforming growth factor-β production, amelioration or prevention of wrinkle, improvement or sustenance of skin elasticity, amelioration or prevention of epidermal thickening, and suppression for decreasing collagen or enhancement for collagen production, characterized in that the cosmetics comprise as an effective ingredient one or more members selected from the group consisting of a fucoidan, a degradation product thereof and a salt thereof. In the present specification, the subject matter of the second invention of the present invention may be simply referred to as "cosmetics" in some cases.

A third invention of the present invention relates to a method selected from a method for enhancement of transforming growth factor-β production, a method for amelioration or prevention of wrinkle, a method for improvement or sustenance of skin elasticity, a method for amelioration or prevention of epidermal thickening, and a method for suppression for decreasing collagen or enhancement of collagen production, characterized by administering to an individual one or more members selected from the group consisting of a fucoidan, a degradation product thereof and a salt thereof.

A fourth invention of the present invention relates to use of one or more members selected from the group consisting of a fucoidan, a degradation product thereof and a salt thereof, for manufacturing a medicament for any one of enhancement of transforming growth factor-β production, amelioration or prevention of wrinkle, improvement or sustenance of skin elasticity, amelioration or prevention of epidermal thickening, and suppression for decreasing collagen or enhancement of collagen production.

### BRIEF DESCRIPTION OF THE DRAWINGS

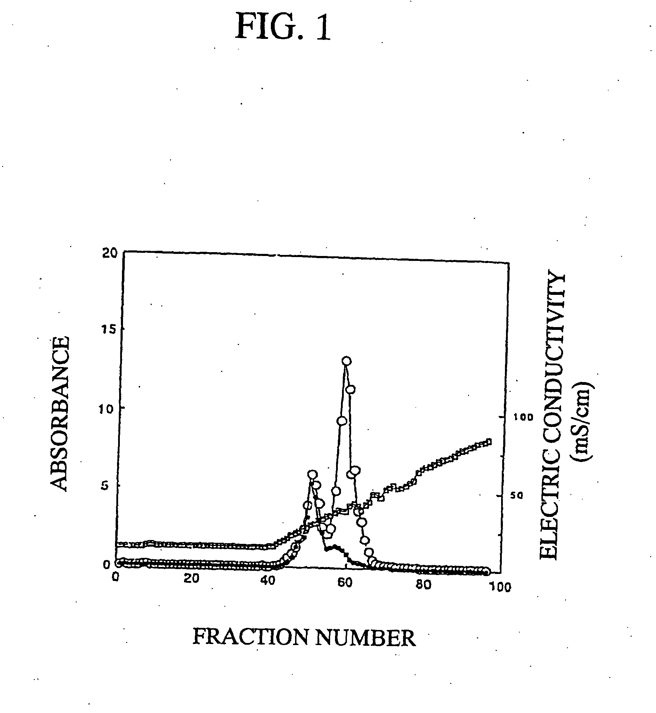

Figure 1 is a graph showing an elution pattern of the fucoidan derived from *Kjellmaniella crassifolia* on DEAE-Cellulofine A-800 column.

### BEST MODE FOR CARRYING OUT THE INVENTION

The medicament and the cosmetics of the present invention is characterized in that the medicament and the cosmetics each comprises as an effective ingredient one or more members selected from the group consisting of a fucoidan, a degradation product thereof and a salt thereof. The exhibition of the desired effects of the present invention is based on an enhancing action of transforming growth factor-β (TGF-β) production, an ameliorative action or prophylactic action of wrinkle, an improving action or sustaining action of skin elasticity, an ameliorative action or prophylactic action of epidermal thickening, and/or a suppressive action for decreasing collagen or an enhancing action for collagen production, which is exhibited by the effective ingredient (these actions of the effective ingredient may be hereinafter simply referred to as "physiological actions."). The action of the effective ingredient was found by the inventors.

The term "enhance" as referred to herein encompasses an embodiment in which the amount of the desired substance is increased after the action of the effective ingredient according to the present invention as compared to that before the action, as well as an embodiment in which the desired substance is produced by the action of the effective ingredient according to the present invention (induce).

The fucoidan which is used as the effective ingredient in the present invention refers to a sulfated fucose-containing polysaccharide, wherein a sulfated fucose is contained as a constituent. The fucoidan is not particularly limited, as long as the fucoidan has the above-mentioned physiological actions. The preferable materials for the fucoidan are exemplified by algae, for instance, marine algae belonging to Phaeophycae such as Laminariales, Chordariales and Fucales, including *Kjellmaniella crassifolia, Laminaria japonica, Kjellmaniella gyrata, Undaria, Ecklonia kurome, Eisenia bicyclis, Ecklonia cava*, Giant kelp, *Lessonia nigrescence, Nemacystus, Cladosiphon okamuranus, Ascophyllum nodosum, Ecklonia maxima* and *Durvillea*; Echinodermata such as sea cucumber, Echnoidea, Asterozoa, and the like. Among them, marine algae of Laminariales richly contain fucoidans which have excellent enhancing actions for TGF-β production and are suitable as the raw materials. Therefore, those derived from algae and those derived from Echinodermata are preferable as the fucoidan which is used as the effective ingredient in the present invention, and those derived from Phaeophycae are more preferable.

These fucoidans may be each prepared by a known method. There can be used in the present invention a crude product, a purified product of a fucoidan, a fucoidan separated into several molecular species, and the like.

For instance, a fucoidan is prepared from *Kjellmaniella crassifolia*, and the resulting fucoidan can be further separated into glucuronic acid-containing fucoidan (referred to as "U-fucoidan") and glucuronic acid non-containing fucoidan (referred to as "F-fucoidan"). Each of these fucoidans can be used as an effective ingredient of the present invention. Also, sulfated fucogalactan (referred to as "G-fucoidan") can be prepared from *Kjellmaniella crassifolia* and used.

For instance, after the preparation of the fucoidans from *Kjellmaniella crassifolia*, U-fucoidan and F-fucoidan are separated by using an anionic exchange resin, a surfactant or the like. The existing ratio of U-fucoidan to F-fucoidan derived from *Kjellmaniella crassifolia* is about 1:2. U-fucoidan contains fucose, mannose, galactose, glucuronic acid and the like, and its sulfate content is about 20%. F-fucoidan contains fucose and galactose, and its sulfate content is about 50%. The molecular weight for both substances is distributed, centering about 200000 (*Summary of 18th Sugar Symposium*, p. 159, 1996).

U-fucoidan and F-fucoidan can be separated, for instance, by applying a fucoidan solution prepared from *Kjellmaniella crassifolia* onto DEAE-Cellulofine A-800 column, and carrying out elution by the concentration gradient technique using NaCl-containing buffer. One of the examples is shown in Figure 1. Concretely, Figure 1 is a diagram showing the separation of U-fucoidan and F-fucoidan, wherein the former peak in the figure is U-fucoidan, and the latter peak is F-fucoidan.

The sea cucumber containing the fucoidan includes, for instance, sea cucumbers described in Japanese Patent Laid-Open No. Hei 4-91027. The fucoidan can be prepared from sea cucumbers in accordance with the method described in the publication. Also, commercially available fucoidans can be used.

The degradation product of the fucoidan used as the effective ingredient according to the present invention (hereinafter may also be referred to as "degradation product according to the present invention") can be prepared by degrading the fucoidan with a known method such as an enzymological method, a chemical method, or a physical method, and selecting a desired degradation product using as an index an enhancing action of TGF-β production, an ameliorative action or prophylactic action of wrinkle, an improving action or sustaining action of skin elasticity, an ameliorative action or prophylactic action of epidermal thickening, and/or a suppressive action for decreasing collagen or an enhancing action for collagen production.

The preferred method for preparing the degradation product of the fucoidan used in the present invention includes, for instance, an acid degradation method. By subjecting the fucoidan to an acid degradation, there can be prepared a degradation product having the above-mentioned physiological actions.

The conditions for the acid degradation of the fucoidan used in the present invention are not particularly limited, as long as the conditions allow to generate the degradation product according to the present invention. For instance, the fucoidan is dissolved or suspended in an acidic aqueous solution or the like and subjected to the acid degradation reaction, whereby a degradation product according to the present invention can be obtained. Also, the reaction mixture may be heated during the reaction, thereby shortening the time period required for the generation of the degradation product according to the present invention. The kinds of the acids for dissolving or suspending the fucoidan are not particularly limited. There can be used an inorganic acid such as hydrochloric acid, sulfuric acid or nitric acid; an organic acid such as citric acid, formic acid, acetic acid, lactic acid, malic acid or ascorbic acid; and a solid acid such as cationic exchange resin, cationic exchange fiber or cationic exchange membrane.

The concentration of the acid is not particularly limited, and the acid can be used at a concentration of preferably from 0.0001 to 5 N or so, more preferably from 0.01 to 1 N or so. In addition, the reaction temperature is not particularly limited, and the reaction temperature may be set at preferably from 0° to 200°C, more preferably from 20° to 130°C.

In addition, the reaction time is not particularly limited, and the reaction time may be set at preferably from several seconds to several days. The kinds and the concentration of the acids, the reaction temperature, and the reaction time may be properly selected depending upon the generated amount of the degradation product according to the present invention and the degree of polymerization of the degradation product. For instance, during the manufacture of the degradation product according to the present invention, an organic acid such as citric acid, lactic acid or malic acid is used, and the concentration of the acid from the range of several dozens mM to several M, the heating temperature from the range of 50° to 110°C, more preferably 70° to 95°C, and the heating time from the range of several minutes to 24 hours are properly selected, whereby the degradation product according to the present invention can be prepared. The acid degradation product of the fucoidan is exemplified by the acid degradation product of the fucoidan derived from *Kjellmaniella crassifolia*, and this degradation product can also be used as dietary fiber having new physiological functions of a strong enhancing action of TGF-β production, ameliorative action or prophylactic action of wrinkle, improving action or sustaining action of skin elasticity, ameliorative action or prophylactic action of epidermal thickening, and/or suppressive action for decreasing collagen or enhancing action for collagen production.

The degradation product according to the present invention can be further fractionated using as an index an enhancing action of TGF-β production, an ameliorative action or prophylactic action of wrinkle, an improving action or sustaining action of skin elasticity, an ameliorative action or prophylactic action of epidermal thickening, and/or a suppressive action for decreasing collagen or an enhancing action for collagen production. For instance, the acid degradation product can be fractionated based on a molecular weight by means of a gel filtration method, a fractionation method using a molecular weight fractionation membrane, or the like.

As an example of gel filtration method, Cellulofine GCL-300 can be used to prepare any molecular weight fractions, for instance, one having a molecular weight exceeding 25000, one having a molecular weight of 25000 to exceeding 10000, one having a molecular weight of 10000 to exceeding 5000, one having a molecular weight of 5000 or less. Cellulofine GCL-25 can be used to prepare any molecular weight fractions from the fraction having a molecular weight of 5000 or less, for instance, one having a molecular weight of 5000 to exceeding 3000, one having a molecular weight of 3000 to exceeding 2000, one having a molecular weight of 2000 to exceeding 1000, one having a molecular weight of 1000 to exceeding 500, one having a molecular weight of 500 or less.

In addition, the degradation product according to the present invention is subjected to industrial molecular weight fractionation using an ultrafiltration membrane. For instance, a fraction having a molecular weight of 30000 or less can be prepared by using FE10-FUS0382 manufactured by DAICEL CHEMICAL INDUSTRIES, LTD., or a fraction having a molecular weight of 6000 or less can be prepared by using FE-FUS-T653 manufactured by the same. Further, a fraction having a molecular weight of 500 or less can be obtained by using a nanofilter membrane. Any molecular weight fractions can be prepared by combining these gel filtration methods and molecular weight fractionation methods.

The degradation product of the fucoidan having the above-mentioned physiological actions which can be used in the present invention is exemplified by the compounds represented by the following formulas (I) to (IV), and these compounds can be prepared in accordance with the methods disclosed in WO 97/26896, WO 99/41288 and WO 00/50464. In addition, the degradation product according to the present invention is exemplified by degradation products of fucoidan described in WO 97/26896, WO 99/41288 and WO 00/50464. 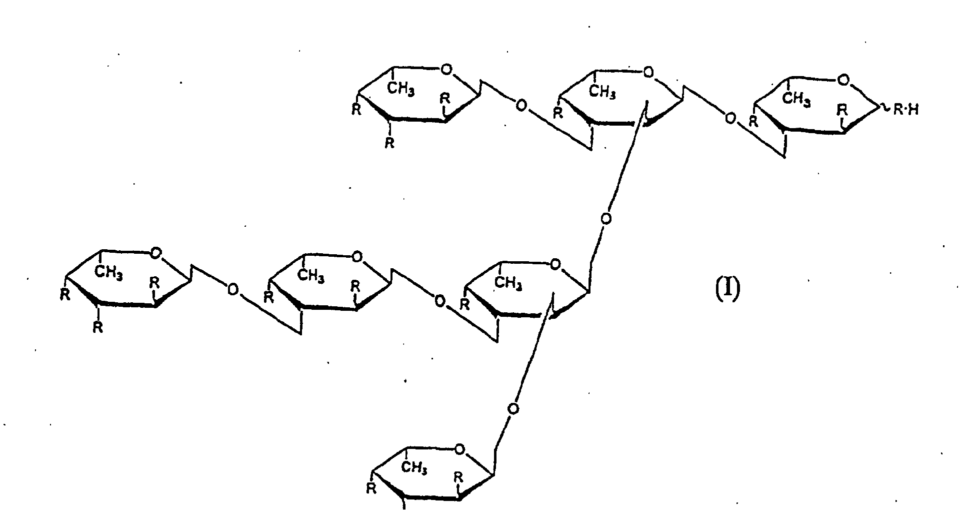 wherein R is OH or OSO₃H; 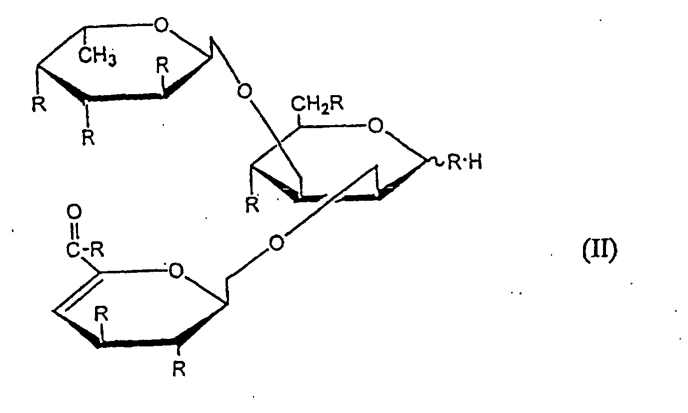 wherein R is OH or OSO₃H; 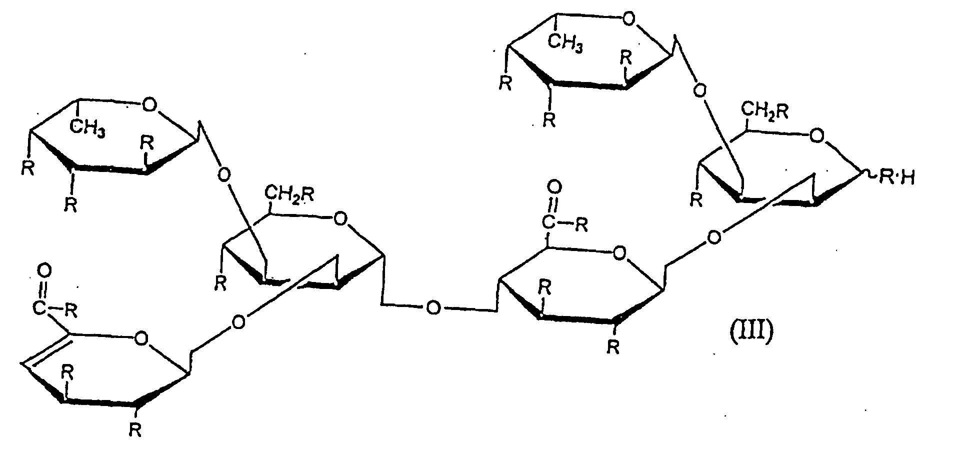 wherein R is OH or OSO₃H; 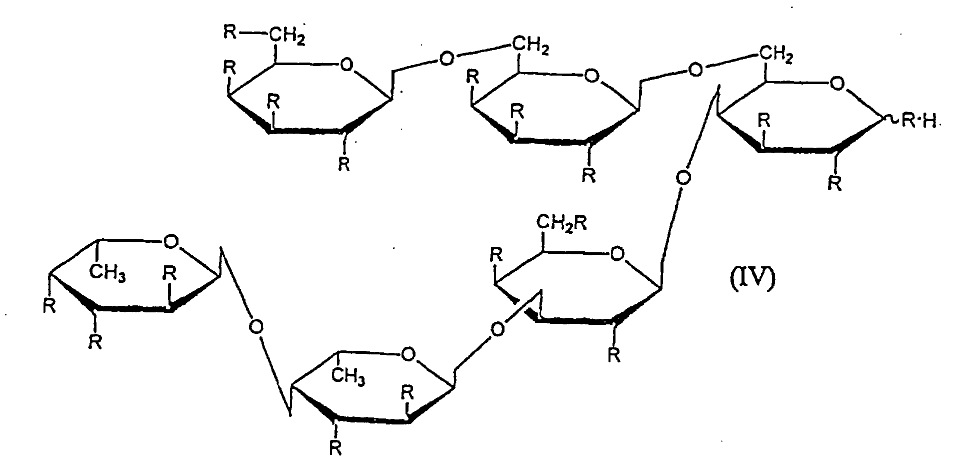 wherein R is OH or OSO₃H.

Examples of the compound represented by the formula (I) include the compound represented by the formula (V) given later. Examples of the compound represented by the formula (II) include the compound represented by the formula (VI) and the formula (VII) given later. Examples of the compound represented by the formula (III) include the compound represented by the formula (VIII).

The compound represented by the formula (I) can be obtained by, for instance, degrading the above-mentioned F-fucoidan with endo-sulfated polysaccharide degrading enzyme (F-fucoidan-specific degradation enzyme) produced by *Alteromonas sp.* SN-1009 (FERM BP-5747), and purifying the resulting degradation product. As to the content and the site of sulfate group in the compound, any ones can be purified from the degradation products. In addition, the polymer of the compound represented by the formula (I) is contained in the degradation products, and can be separated and purified depending on its purposes.

Each of the compounds represented by the formula (II) and the formula (III) can be obtained by, for instance, degrading the above-mentioned U-fucoidan with endo-sulfated polysaccharide degrading enzyme (U-fucoidan-specific degradation enzyme) produced by *Flavobacterium sp.* SA-0082 (FERM BP-5402), and purifying the resulting degradation products. As to the content and the site of sulfate group in the compound, any ones can be purified from the degradation products. In addition, the polymer of which basic backbone structure comprises the compound represented by the formula (II) or the formula (III) is also contained in the degradation products, and can be separated and purified depending on its purposes.

In addition, the above-mentioned G-fucoidan can be prepared by degrading the fucoidan derived from *kjellmaniella crassifolia* with F-fucoidan-specific degradation enzyme produced by *Alteromonas sp.* SN-1009 (FERM BP-5747) and U-fucoidan-specific degradation enzyme produced by *Flavobacterium sp.* SA-0082 (FERM BP-5402), and purifying the resulting degradation product.

The above-mentioned *Flavobacterium sp.* SA-0082 also produces endo-sulfated polysaccharide degrading enzyme, which specifically degrades G-fucoidan (G-fucoidan-specific degradation enzyme). The G-fucoidan is further treated with the G-fucoidan-specific degrading enzyme to prepare a degradation product of the G-fucoidan, and the degradation product is purified according to its purpose, whereby a degradation product which can be used as an effective ingredient according to the present invention can be prepared from the above degradation product. The compound represented by the formula (IV) is one such example. As to the content and the site of sulfate group in the compound, any ones can be purified from the degradation products. In addition, the polymer of which basic backbone structure comprises the compound represented by the formula (IV) is also contained in the above degradation products, and can be separated and purified according to its purposes.

Each of the above-mentioned enzymes is described in WO 97/26896 and WO 00/50464.

As described in item (3) of Preparation Example 2 described later, a highly sulfated product in which the fucoidan or a degradation product thereof is further added with sulfate group can be also used as an effective ingredient in the present invention.

The salt of the fucoidan or a degradation product thereof used as an effective ingredient according to the present invention is exemplified by, for instance, alkali metal salts, alkaline earth metal salts, and salts with organic bases. Examples thereof include salts with sodium, potassium, calcium, magnesium, ammonium, diethanolamine, ethylenediamine, and the like. These salts are obtained by converting sulfate group or carboxyl group existing in fucoidans and the like to salts according to a known method. The salt is preferably a pharmacologically acceptable salt.

The exhibition of the physiological function of the effective ingredient according to the present invention can be evaluated by the method described in Examples described later. For instance, the enhancing action for TGF-β production can be evaluated by the method described in Example 1, and the ameliorative action or prophylactic action of wrinkle, the improving action or sustaining action of skin elasticity, the ameliorative action or prophylactic action of epidermal thickening, and/or the suppressive action for decreasing collagen or enhancing action for collagen production can be evaluated by the methods described in Example 5, 6, 8, 9 and/or 10. The amount of type I collagen produced in a cell reflects the produced amount of type I pro-collagen, which is a precursor of type I collagen. In other words, the changes in the production of type I collagen can be known by examining the amount of the type I pro-collagen produced.

The medicament, which is the first embodiment of the present invention, comprises the above-mentioned effective ingredient according to the present invention, which is provided as a therapeutic agent or prophylactic agent for a disease requiring enhancement of TGF-β production, an agent for amelioration or prevention of wrinkle, an agent for improvement or sustenance of skin elasticity, an agent for amelioration or prevention of epidermal thickening, or a suppressor for decreasing collagen or an enhancer for collagen production.

The transforming growth factor (TGF) exists in α-form (TGF-α) and β-form (TGF-β). Especially, TGF-β has a variety of physiological activities such as suppression of cell proliferation, acceleration of cell differentiation, suppression of immunological functions and facilitation of motility of fibroblasts, and is considered to be associated with curing various diseases. For instance, there have been known effects for treating or preventing diabetic retinopathy, initial lesion of atherosclerosis, bone fracture, cardiac infarction, cardiac infarction after ischemic reperfusion, cerebral infarction, retinodialysis, or the like, an effect for healing a wound, and the like. Therefore, there can be expected an effect of treating or preventing these diseases by the enhancement of TGF-β production.

In other words, in the present invention, a diseases requiring enhancement of TGF-β production refers to a disease which can be treated or prevented by the enhancement of TGF-β production. The disease is exemplified by, for instance, diabetic retinopathy, atherosclerosis, bone fracture, cardiac infarction, cardiac infarction after ischemic reperfusion, cerebral infarction, retinodialysis, wound and the like. The effective ingredient according to the present invention has an enhancing action for TGF-β production, and the therapeutic agent or prophylactic agent of the present invention comprising the effective ingredient is effective for treating or preventing the disease. Incidentally, TGF-β exists in various isoforms. Among those isoforms of TGF-β, a fucoidan, a degradation product thereof and/or a salt thereof which is used as the effective ingredient according to the present invention has an especially excellent enhancing action for TGF-β₁ production. The TGF-β which is enhanced for production by the effective ingredient is preferably exemplified by TGF-β₁.

In addition, the effective ingredient according to the present invention has an ameliorative action or prophylactic action of wrinkle, an improving action or sustaining action of skin elasticity, an ameliorative action or prophylactic action of epidermal thickening, and/or a suppressive action for decreasing collagen or an enhancing action for collagen production. Therefore, the agent for amelioration or prevention of wrinkle, the agent for improvement or sustenance of skin elasticity, the agent for amelioration or prevention of epidermal thickening, or the suppressor for decreasing collagen or an enhancer for collagen production according to the present invention, each of which comprises the effective ingredient, exhibits excellent effects such as amelioration or prevention of wrinkle. For instance, by applying to skin an agent for amelioration or prevention of wrinkle, an agent for improvement or sustenance of skin elasticity, an agent for amelioration or prevention of epidermal thickening, or a suppressor for decreasing collagen or an enhancer for collagen production according to the present invention, any of wrinkle formation, lowering of skin elasticity, increase in epidermal thickening or decrease in collagen, which are induced by an external factor such as exposure to sun light, ultraviolet ray irradiation or drying, or an internal factor for causing aging phenomena, can be also ameliorated or prevented. In the present invention, the term "amelioration of wrinkle" means making the length of wrinkle shorter, the depth of wrinkle shallower, or reducing to no wrinkles.

Subsequently, the process for manufacturing a medicament of the present invention will be explained. The medicament of the present invention comprises as an effective ingredient one or more members selected from the group consisting of a fucoidan, a degradation product thereof and a salt thereof, and is obtained by combining the effective ingredient with a known medicinal vehicle to make a preparation. The medicament is generally produced by formulating the effective ingredient according to the present invention with a pharmacologically acceptable liquid or solid vehicle, and optionally adding a solvent, a dispersant, an emulsifier, a buffer, a stabilizer, an excipient, a binder, a disintegrant, a lubricant, or the like, and forming the resulting mixture into a solid agent such as a tablet, a granule, a powder, a fine powder, or a capsule, or a liquid agent such as a general liquid agent, a suspension agent, or an emulsion agent. In addition, the medicament can be formed into a dry product which can be made into a liquid form by adding an appropriate vehicle immediately before use, or into external preparation.

The medicinal vehicle can be selected depending upon the above-mentioned administration form and preparation form of the medicament of the present invention. In the case of an orally administered preparation, there can be utilized, for instance, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a flavor, a colorant, a perfume, and the like can be further formulated.

On the other hand, a non-orally administered preparation can be prepared by dissolving or suspending the effective ingredient according to the present invention, in a diluent such as distilled water for injection, physiological saline, an aqueous solution of glucose, vegetable oil for injection, sesame oil, peanut oil, soybean oil, corn oil, propylene glycol or polyethylene glycol, according to a conventional method, and adding optionally a microbicide, a stabilizer, an osmotic regulator, a soothing agent, or the like.

External preparation includes solid, hemi-solid or liquid preparation for percutaneous administration. The external preparation also includes suppositories and the like. For instance, the external preparation may be prepared as liquid preparations including emulsions, suspensions such as lotions and external tinctures; ointments including oily ointments and hydrophilic ointments; medical adhesives for percutaneous administration such as films, tapes and poultices; and the like.

The medicament can be appropriately manufactured by known methods in the field of pharmaceuticals. The content of the effective ingredient according to the present invention in the medicament of the present invention is not particularly limited, as long as the content is preferably the amount so that the effective ingredient can be administered within the dose described later in consideration of administration form, administration method and the like of the medicament.

The medicament of the present invention can be administered via an administration route appropriate for each of the preparation form. The administration method is not limited to specific one. The medicament can be administered internally, externally (or topically) and by injection. It is especially preferable that the agent for amelioration or prevention of wrinkle, the agent for improvement or sustenance of skin elasticity, the agent for amelioration or prevention of epidermal thickening, or the suppressor for decreasing collagen or the enhancer for collagen production according to the present invention is administered by applying to skin as the above-mentioned external preparation, whereby the desired effect, for instance, an effect for amelioration or prevention of wrinkle, can be obtained. The injection can be administered, for instance, intravenously, intramuscularly, subcutaneously, intracutaneously, or the like.

The dose of the medicament of the present invention is changeable and properly set depending upon its preparation form, administration method, purpose of use, and age, body weight, or symptom of a patient to whom the medicament is applied, and the like. Generally, the dose of the above-mentioned effective ingredient contained in the preparation is preferably from 0.1 to 2000 mg/kg per day for adult. As a matter of course, the dose varies depending upon various conditions, so that an amount smaller than the dose mentioned above may be sufficient, or an amount exceeding the dose range may be required. Administration may be carried out once or several divided portions in a day within the desired dose range. Also, the medicament of the present invention can not only be orally administered *per se* but also be taken on a daily basis by adding the medicament to optional foodstuff. In addition, the fucoidan, a degradation product thereof and/or a salt thereof may be used as raw materials for foodstuff for enhancement of TGF-β production, amelioration or prevention of wrinkle, improvement or sustenance of skin elasticity, amelioration or prevention of epidermal thickening, and/or suppression for decreasing collagen or enhancement of collagen production.

In addition, due to its physiological actions, the fucoidan, a degradation product thereof and/or a salt thereof which is used as an effective ingredient according to the present invention is useful for functional studies on TGF-β, for studies on mechanisms of wrinkle formation, lowering of skin elasticity, epidermal thickening and decreasing collagen, and for screening of not only the medicament of the present invention but also for a medicament for a disease associated with various TGF-β, an agent for amelioration or prevention of wrinkle, an agent for improvement or sustenance of skin elasticity, an agent for amelioration or prevention of epidermal thickening, and a suppressor for decreasing collagen or an enhancer for collagen production.

The cosmetics, a second embodiment of the present invention, comprise the above-mentioned effective ingredient according to the present invention, and is provided as cosmetics for enhancement of TGF-β production, cosmetics for amelioration or prevention of wrinkle, cosmetics for improvement or sustenance of skin elasticity, cosmetics for amelioration or prevention of epidermal thickening, and cosmetics for suppression for decreasing collagen or enhancement of collagen production. The desired effects of the cosmetics of the present invention are exhibited on the basis of the above-mentioned physiological actions owned by the effective ingredient contained in the cosmetics. Also, it is presumed that if the TGF-β production is enhanced in the internal of the skin, the stretch and elasticity of skin can be increased, so that the enhancing action for TGF-β production is thought to synergistically act on the ameliorative action or prophylactic action of wrinkle, or the like. Therefore, according to the cosmetics of the present invention, stretch or elasticity of skin, for instance, can be effectively improved. In other words, according to the present invention, there are provided cosmetics having an excellent enhancing action for TGF-β production, an ameliorative action or prophylactic action of wrinkle, an improving action or sustaining action of skin elasticity, an ameliorative action or prophylactic action of epidermal thickening, or a suppressive action for decreasing collagen or enhancing action for collagen production, each of which comprises as an effective ingredient one or more members selected from the group consisting of a fucoidan, a degradation product thereof and a salt thereof.

Here, the fucoidan which is used as the effective ingredient is preferably derived from algae or derived from Echinodermata, and the algae is preferably Phaephycae. In addition, the degradation product of the fucoidan is not particularly limited. The compound selected from the compounds represented the above-mentioned formulas (I) to (IV) can be preferably used.

The content of the effective ingredient according to the present invention in the cosmetics of the present invention is usually preferably from 0.0001 to 20% by weight, more preferably from 0.001 to 5% by weight, still more preferably from 0.03 to 3% by weight.

In the cosmetics of the present invention, there can be contained, as other components besides the effective ingredient according to the present invention, moisturizing agents such as 1,3-butylene glycol and pyrrolidonecarboxylates; skin unlimbering agents such as liquid paraffins, vaseline, olive oil, squalene, lanoline and synthetic ester oils; fats and oils such as coconut oil and palm oil; vitamins such as vitamin E; surfactants such as beeswax, propylene glycol monostearate and stearic acid; emulsification stabilizing aids such as stearyl alcohol; solubilizing agents such as polyoxyethylene cetyl ether and polyoxyethylene cured castor oil; anticorrosive agents such as methyl p-benzoate (methylparaben); pigments; antioxidants; ultraviolet absorbents; pharmacologically active substances; base materials; surfactants; and the like. Furthermore, one having moisture-retaining action such as glycerol, propylene glycol, polyethylene glycol, or polysaccharides represented by hyaluronic acid may be used together therewith.

The form of the cosmetics is not particularly limited, as long as the above-mentioned physiological actions of the effective ingredient can be expected, and the preferable form includes, for instance, a lotion, a milky lotion, cream, a facial pack, a bathing agent, a facial cleansing agent, a bathing soap, a bath detergent and an ointment.

The cosmetics of the present invention can be appropriately prepared in accordance with a method known in the field of cosmetics using the effective ingredient according to the present invention and the above-mentioned other ingredients as desired as the raw materials. In addition, for instance, cosmetics which are suitable for oral intake can be manufactured in accordance with a method known in the field of foods and beverages in the same manner as the food, beverage and the like described later.

For instance, the cosmetics containing the effective ingredient according to the present invention in the above-mentioned content range are applied in a desired amount depending upon its application form. For instance, if the cosmetics are a lotion, which is applied to, for instance, an entire facial surface of human, the lotion is used in an amount of preferably from 0.01 to 5 g or so, more preferably from 0.1 to 2 g or so, per use. Consequently, there is obtained an enhancing effect for TGF-β production, an ameliorative effect or prophylactic effect of wrinkle, an improving effect or sustaining effect of skin elasticity, an ameliorative effect or prophylactic effect of epidermal thickening and/or a suppressive effect for decreasing collagen or enhancing effect for collagen production, whereby giving desired effects of the present invention such as an effect of giving stretch and gloss to skin and obtaining lustrous skin.

In addition, when the cosmetics are suitable for oral intake, the effective amount for oral intake of the cosmetics is not particularly limited as long as the effective amount according to the present invention can be taken in an amount which can show its physiological actions. For instance, the effective amount is preferably from 0.1 mg to 10 g, more preferably from 2 mg to 5 g, still more preferably from 10 mg to 2 g, per day for adult.

The skin as referred to herein includes all parts covering the outside of human or an animal, such as face, neck, breast, back, arm, hand, leg, foot and scalp.

In addition, a third embodiment of the present invention provides a method for enhancement of TGF-β production, a method for amelioration or prevention of wrinkle, a method for improvement or sustenance of skin elasticity, a method for amelioration or prevention of epidermal thickening, or a method for suppression for decreasing collagen or enhancement of collagen production, characterized by administering to an individual one or more members selected from the group consisting of a fucoidan, a degradation product thereof and a salt thereof.

The fucoidan, a degradation product thereof and a salt thereof used in the embodiment of the present invention are the same as the above-mentioned effective ingredient according to the present invention, and the fucoidan to be administered to an individual is preferably derived from algae or derived from Echinodermata. The algae is preferably Phaeophycae. The term "individual" as referred to herein is a mammal, preferably human.

The method can be carried out by administering the above-mentioned effective ingredient, preferably as the therapeutic agent or prophylactic agent of the present invention, to human who is expected to need, for instance, the enhancement of TGF-β production, or who requires such enhancement, whereby the TGF-β production is enhanced. As a result, there can be expected an effect for treating or preventing a disease requiring, for instance, enhancement of TGF-β production. In addition, the method can be carried out by administering the above-mentioned effective ingredient preferably as the agent for amelioration or prevention of wrinkle, the agent for improvement or sustenance of skin elasticity, the agent for amelioration or prevention of epidermal thickening, or the suppressor for decreasing collagen or enhancer for collagen production according to the present invention to human who desires to ameliorate or prevent wrinkle or the like, whereby the desired effects such as amelioration or prevention of wrinkle can be obtained. The method for administration, the dose and the like of the effective ingredient of the present invention may be in accordance with the method for administration, the dose of the medicament and the like of the present invention used in administration of the effective ingredient. Further, in the embodiment of the present invention, the above-mentioned cosmetics and the food, beverage or feed mentioned later provided according to the present invention can be also used.

In addition, another embodiment of the present invention provides a food, beverage or feed for enhancement of TGF-β production, amelioration or prevention of wrinkle, improvement or sustenance of skin elasticity, amelioration or prevention of epidermal thickening, and/or suppression for decreasing collagen or enhancement of collagen production, wherein the food, beverage or feed is prepared by containing, adding and/or diluting the above-mentioned effective ingredient according to the present invention. By the physiological action of the effective ingredient, the food, beverage or feed according to the present invention is very useful for amelioration or prevention for a disease requiring enhancement of TGF-β production, amelioration or prevention of wrinkle, improvement or sustenance of skin elasticity, amelioration or prevention of a symptom accompanied with epidermal thickening, and/or amelioration or prevention of a symptom accompanied with decreasing collagen in an individual showing sensitivity to the effective ingredient according to the present invention.

In the embodiment of the present invention, the term "containing" refers to an embodiment of containing the effective ingredient used in the present invention in the food, beverage or feed; the term "adding" refers to an embodiment of adding the effective ingredient used in the present invention to a raw material for the food, beverage or feed; and the term "diluting" refers to an embodiment of diluting the effective ingredient used in the present invention with a raw material for the food, beverage or feed. Also, the term "containing" in the description of the above-mentioned cosmetics of the present invention encompasses "containing," "adding" and "diluting" as referred to in the embodiment of the present invention.

The method for preparing the food or beverage according to the present invention is not particularly limited. The method includes cooking, processing, and any process by which foods or beverages can be generally prepared, as long as the fucoidan, a degradation product thereof and/or a salt thereof used as the effective ingredient according to the present invention is contained, added and/or diluted in the food or beverage prepared.

The food or beverage according to the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham and sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks, and rice snacks; alcohol beverages such as *sake,* Chinese liquor, wine, whisky, Japanese distilled liquor *(shochu),* vodka, brandy, gin, ram, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste (*miso*) soup, precooked foods, precooked beverages, and precooked soup; frozen foods such as *sukiyaki*, pot-steamed hotchpotch, split and grilled eel, hamburger steak, *shao-mai*, dumpling stuffed with minced pork, various sticks, and fruit cocktails; solid foods; liquid foods (soups); spices; and the like.

The content of the effective ingredient according to the present invention in the food or beverage according to the present invention is not particularly limited, and the content can be appropriately selected from the viewpoints of sensory ability and exhibition of the actions. The content of the effective ingredient is, for instance, preferably 0.0001 parts by weight or more, more preferably from 0.001 to 10 parts by weight, per 100 parts by weight of the food, or for instance, preferably 0.0001 parts by weight or more, more preferably from 0.001 to 10 parts by weight, per 100 parts by weight of the beverage.

In addition, according to the present invention, there is provided a feed for an organism prepared by containing, adding and/or diluting the above-mentioned effective ingredient according to the present invention (which may be hereinafter referred to as "the feed of the present invention"). In still another embodiment, the present invention also provides a method of feeding an organism, characterized by administering the above-mentioned effective ingredient to the organism. In still yet another embodiment, the present invention provides an organism feeding agent characterized in that the organism feeding agent comprises the above-mentioned effective ingredient.

In these inventions, the organism includes, for instance, culturing or breeding animals, pet animals, and the like. The culturing or breeding animal is exemplified by cattle, laboratory animals, poultry, pisces, crustaceae or shellfish. The feed is exemplified by a feed for sustenance of and/or improvement in physical conditions. The organism feeding agent is exemplified by immersion agents, feed additives, and beverage additives.

According to these inventions, the same effects can be expected to be exhibited as those of the above-mentioned medicament of the present invention, on the basis of the physiological functions of the effective ingredient according to the present invention in the organism exemplified above for applying these. For instance, there can be obtained an effect for treating or preventing a disease requiring enhancement of TGF-β production, for instance, diabetic retinopathy, atherosclerosis, bone fracture, cardiac infarction, cardiac infarction after ischemic reperfusion, cerebral infarction, or retinodialysis, or an effect for healing a wound, besides the ameliorative effect or prophylactic effect of wrinkle, the improving effect or sustaining effect of skin elasticity, the ameliorative effect or prophylactic effect of epidermal thickening, and/or the suppressive effect for decreasing collagen or enhancing effect for collagen production.

In the feed according to the present invention, the above-mentioned effective ingredient used in the present invention is usually administered in an amount of preferably from 0.01 to 2000 mg per 1 kg of the subject organism per day. The administration can be made using a feed prepared by adding and mixing the effective ingredient in a raw material for an artificially formulated feed, or by mixing the effective ingredient with a powder raw material for an artificially formulated feed, and thereafter further adding and mixing the resulting mixture with other raw materials. In addition, the content of the effective ingredient according to the present invention in the feed for a subject organism is not particularly limited. The effective ingredient can be appropriately set in accordance with its purposes, and an appropriate proportion in the feed is preferably in a ratio of from 0.001 to 15% by weight.

The artificially formulated feed includes feeds using animal-derived raw materials such as fish meal, casein, and squid meal; plant-derived raw materials such as soybean grounds, flour, and starch; microorganism raw materials such as yeasts for feed; animal fats and oils such as cod-liver oil and squid-liver oil; vegetable fats and oils such as soybean oil and rapeseed oil; and vitamins, minerals, amino acids, and antioxidants; and the like as raw materials. In addition, feeds for fish such as fish minced meat are also included.

The method for preparing the feed according to the present invention is not particularly limited. The feed can be prepared according to a general method for a feed, as long as the effective amount of the above-mentioned effective ingredient according to the present invention may be contained, added and/or diluted in the feed produced.

Also, the effective ingredient according to the present invention can be administered by directly adding the effective ingredient to water, seawater, or the like in a pool, a water tank, a water reservoir, or a feeding range, and immersing a subject organism into the resulting solution. The immersion method is especially effective when the amount of intake of the feed of the subject organism is lowered. The concentration of the above-mentioned effective ingredient used in the present invention in water or seawater is not particularly limited, and the concentration may be set in accordance with its purposes. It is appropriate that the concentration is preferably from 0.00001 to 1% by weight.

Also, a beverage comprising the effective ingredient according to the present invention may be given to a subject organism as a feeding drink. The concentration of the effective ingredient according to the present invention in the beverage is not particularly limited, and the concentration may be set in accordance with its purposes. It is appropriate that the concentration is preferably from 0.0001 to 1% by weight. The organism feeding agent, for instance, an immersion agent, a feed additive, or a beverage additive comprising the above-mentioned effective ingredient according to the present invention may be prepared by a known method. The content of the effective ingredient in the organism feeding agent is not particularly limited, so long as the desired effects of the present invention can be obtained.

The organism to which these feeds or the like according to the present invention can be applied is not limited. The culturing or breeding animals include cattle such as *Equus, Bos, Porcus, Ovis, Capra, Camelus*, and *Lama;* laboratory animals such as mice, rats, guinea pigs, and rabbits; poultry such as *Chrysolophus,* ducks, *Meleagris*, and *Struthioniformes;* pisces such as *Pagrus, Oplegnathidae, Paralichthys*, plaice, *Seriola,* young *Seriola*, amberjack, *Thunna, Caranx delicatissimus, Plecoglossus, Saimo*•*Oncorhynchus, Fugu, Anguilla, Misguirus*, and *Parasilurus*; Crustaceae such as *Penaidae*, black tiger shrimp, *Penaeus roentalis*, and *Portulus trituberculatus*; and shellfish such as abalones (*awabi*), turban shells, scallops, and oysters; and the pet animals include dogs, cats, and the like, so that the feed can be widely applied to animals on land and in water.

The method for feeding an organism provided by the present invention, in which the effective ingredient according to the present invention is administered to the organism can be carried out, for instance, by administering the above-mentioned feed and/or organism feeding agent according to the present invention to an organism to be administered so that the desired effects of the present invention can be obtained.

By allowing a subject organism to take the feed according to the present invention, or immersing a subject organism into a solution containing the above-mentioned effective ingredient according to the present invention, the physical conditions of the cattle, experimental animals, poultry, pisces, Crustacea, shellfish, pet animals or the like can be well sustained and ameliorated.

The food, beverage or feed according to the present invention does not have any particular limitation on its shape, as long as the fucoidan, a degradation product thereof and/or a salt thereof used as an effective ingredient according to the present invention is contained therein, added thereto and/or diluted therewith, so that the feed, beverage or feed comprises the effective ingredient in an amount effective for exhibiting its physiological actions. For instance, such shapes include orally taken shapes such as tablets, granules and capsules. Here, the effective ingredient according to the present invention is health food material having both physiological action and dietary fiber function, so that the effective ingredient is extremely useful as a production material for a food, beverage or feed.

A still another embodiment of the present invention provides use of one or more members selected from the group consisting of a fucoidan, a degradation product thereof and a salt thereof, for manufacturing a medicament for any one of enhancement of TGF-β production, amelioration or prevention of wrinkle, improvement or sustenance of skin elasticity, amelioration or prevention of epidermal thickening, and suppression for decreasing collagen or enhancement of collagen production.

There are no cases of death when a fucoidan, a degradation product thereof and/or a salt thereof used as the effective ingredient in the present invention is orally administered to a mouse once at 1 g/kg. In addition, there are no occurrence of adverse actions.

### EXAMPLES

The present invention will be more concretely described by means of the following examples, without limiting the scope of the present invention thereto. Here, "%" in Examples means "% by weight" unless otherwise indicated.

### Preparation Example 1

(1) *Kjellmaniella crassifolia* was sufficiently dried, and thereafter 20 kg of the dried product was powdered with a free mill (manufactured by Nara Kikai Seisakusho).

In 900 liters of tap water was dissolved 7.3 kg of calcium chloride dihydrate (manufactured by Nippon Soda Co., Ltd.), and 20 kg of the powdered product of *Kjellmaniella crassifolia* was then mixed therewith. The resulting mixture was heated for 40 minutes by blowing steam until the liquid temperature was raised from 12° to 90°C. Thereafter, the mixture was kept at 90° to 95°C for 1 hour under stirring, and then cooled, to give 1100 liters of a cooled product.

Subsequently, the cooled product was subjected to solid-liquid separation with a solid-liquid separator (manufactured by West Farrier Separator, Model: CNA), to give about 900 liters of supernatant after solid-liquid separation.

The amount 360 liters of the supernatant after solid-liquid separation was concentrated up to a volume of 20 liters with FE10-FC-FUS0382 (fraction molecular weight: 30000) manufactured by DAICEL CHEMICAL INDUSTRIES, LTD. Thereafter, the steps of adding 20 liters of tap water and again concentrating the resulting liquid mixture up to a volume of 20 liters were repeated 5 times, and the concentrate was subjected to a desalting treatment, to give 25 liters of an extract derived from *Kjellmaniella crassifolia*.

One liter of the extract was lyophilized, to give 13 g of a dried product of a fucoidan derived from *Kjellmaniella crassifolia*.

(2) Seven grams of the dried product of the fucoidan described in item (1) of Preparation Example 1 was dissolved in 700 mL of a 20 mM imidazole buffer (pH 8) containing 50 mM sodium chloride and 10% ethanol, and insoluble substances were removed by centrifugation. The supernatant after centrifugation was applied onto a DEAE-Cellulofine A-800 column (φ 11.4 cm x 48 cm) (manufactured by Seikagaku Corporation) equilibrated with the same buffer, and then washed with the same buffer. The elution was carried out with a concentration gradient of from 50 mM to 1.95 M sodium chloride (250 mL per fraction). A total sugar content and an uronic acid content were determined by the phenol-sulfuric acid method and the carbazole-sulfuric acid method, to give Fractions 43 to 49, Fractions 50 to 55, and Fractions 56 to 67, in the order of elution. Next, these fractions were desalted by electrodialysis, and thereafter lyophilized, to give each of Fraction I (340 mg) from Fractions 43 to 49, Fraction II (870 mg) from Fractions 50 to 55, and Fraction III (2.64 g) from Fractions 56 to 67.

Figure 1 shows an elution pattern of the fucoidan derived from *Kjellmaniella crassifolia* on the DEAE-Cellulofine A-800 column. In Figure 1, the axis of ordinates is the absorbance at 530 nm as determined by the carbazole-sulfuric acid method (solid circles in the figure), the absorbance at 480 nm as determined by the phenol-sulfuric acid method (open circles in the figure), and the electric conductivity (mS/cm: open squares in the figure), and the axis of abscissas is the fraction number. In the figure, the front peak shows U-fucoidan, and the back peak shows F-fucoidan.

(3) A sulfated fucose-containing polysaccharide fraction was prepared from dried *Kjellmaniella crassifolia*. Specifically, 2 kg of commercially available dried *Kjellmaniella crassifolia* was powdered with a cutter mill (manufactured by Masuko Sangyo) fitted with a screen having a hole diameter of 1 mm, and the resulting powders were suspended in 20 liters of 80% ethanol. The suspension was stirred at 25°C for 3 hours and filtered with a filter paper. The residue obtained was suspended in 40 liters of a 30 mM sodium phosphate buffer containing 100 mM sodium chloride (pH 6.5), the suspension was allowed to stand at 95°C for 2 hours. Thereafter, the suspension was filtered with a stainless screen having a hole diameter of 106 µm. To this filtrate were added 200 g of activated carbon, 4.5 liters of ethanol, and 12000 U of alginic acid lyase K (manufactured by Nagase Seikagaku Kogyo), and the mixture was stirred at 25°C for 20 hours and then centrifuged. The resulting supernatant was concentrated to a volume of 4 liters with an ultrafilter equipped with holofiber having an excluding molecular weight of 100000, and thereafter insoluble substances were removed by centrifugation. The resulting solution was allowed to stand at 5°C for 24 hours, and precipitates formed were removed by centrifugation. The solvent for the resulting supernatant was exchanged for 100 mM sodium chloride with an ultrafilter. This solution was cooled to 4°C or lower, thereafter the pH of the solution was adjusted to 2 with hydrochloric acid, and precipitates formed were removed by centrifugation. The pH of the resulting supernatant was adjusted to 8 with sodium hydroxide, and the resulting solution was concentrated to a volume of 4 liters. Thereafter, the solvent for the resulting solution was exchanged for 20 mM sodium chloride with an ultrafilter. After insoluble substances in this solution were removed by centrifugation, the resulting supernatant was lyophilized, to give 76 g of a dried product of a fucoidan derived from *Kjellmaniella crassifolia*. In addition, the same procedures as in the above were carried out, to prepare each of a fucoidan derived from *Laminaria japonica*, a fucoidan derived from *Lessonia nigrescence*, a fucoidan derived from *Ecklonia maxima*, and a fucoidan derived from *Durvilliae*.

(4) Five-hundred grams of dried *Kjellmaniella crassifolia* was cut into thin pieces, and washed with 10 liters of 80% ethanol. Thereafter, the resulting product was stirred in 50 liters of 10% ethanol containing 1 mM potassium chloride at 25°C for 3 days, and filtered with a stainless screen having a screen diameter of 32 µm, to give about 45 liters of an F-fucoidan-containing filtrate.

(5) Five-hundred grams of dried *Kjellmaniella crassifolia* was cut into thin pieces, and washed with 10 liters of 80% ethanol. Thereafter, the resulting product was stirred at 25°C for 3 days in 50 liters of 10% ethanol containing 1 mM potassium chloride, and filtered with a stainless screen having a screen diameter of 32 µm, to give about 45 liters of a filtrate. Thirty-four liters of this filtrate was heated at 80°C for 3 hours, and thereafter cooled to 50°C. The resulting solution was concentrated with an ultrafilter OMEGA Cassette (manufactured by Filtron) having an excluding molecular weight of 10000 with keeping the liquid temperature at 50°C. Further, the concentrate was desalted with 5 liters of distilled water heated to 50°C, and the flow path was washed twice by adding 200 mL of the same distilled water, and washings were collected, to give 1.5 liters of a concentrate. This concentrate was lyophilized, to give 8.2 g of F-rich fucoidan. Incidentally, F-rich fucoidan refers to a fucoidan comprising a sulfated fucose as a major constituent of the constituting saccharide.

### Preparation Example 2

(1) A 2-liter Erlenmeyer flask was charged with 600 mL of a culture medium comprising an artificial sea water (manufactured by Jamarin Laboratory), pH 8.2, containing 0.25% glucose, 1.0% peptone, and 0.05% yeast extract, and then sterilized (at 120°C for 20 minutes). *Alteromonas* sp. SN-1009 (FERM BP-5747) was inoculated into the culture medium, and cultured at 25°C for 26 hours, to give a seed culture medium. A 30-liter jar fermentor was charged with 20 liters of a culture medium comprising an artificial sea water, pH 8, containing 1.0% peptone, 0.02% yeast extract, 0.2% sulfated polysaccharide described in item (2) of Preparation Example 2 described below, and 0.01% defoaming agent (manufactured by Shin-Etsu Chemical Co., Ltd., KM70), and sterilized at 120°C for 20 minutes. After cooling, a 30 L jar fermentor was charged with 600 mL of the above-mentioned seed culture medium, and cultured at 24°C for 24 hours under the conditions of 10 liters of aeration per minute and a stirring rate of 250 rpm. After termination of the culture, the culture medium was centrifuged, to give cells and culture supernatant. This culture supernatant was concentrated with an ultrafilter equipped with holofiber having an excluding molecular weight of 10000, and the concentrate was then subjected to salting out with an 85% saturated ammonium sulfate. Precipitates formed were harvested by centrifugation, and sufficiently dialyzed against a 20 mM Tris-HCl buffer (pH 8.2) containing an artificial sea water at a one-tenth concentration, to give 600 mL of a solution of an endo-sulfated polysaccharide-degrading enzyme (F-fucoidan-specific degradation enzyme), selectively acting on the sulfated polysaccharide.

(2) Two kilograms of dried *Kjellmaniella crassifolia* was powdered with a cutter mill fitted with a screen having a diameter of 1 mm, and the resulting seaweed chips were suspended in 20 liters of 80% ethanol. The suspension was stirred at 25°C for 3 hours and filtered with a filter paper, and thereafter the residue was sufficiently washed. The residue obtained was suspended in 20 liters of a 50 mM imidazole buffer (pH 8.2) containing 30 mL of F-fucoidan-specific degradation enzyme prepared in item (1) of Preparation Example 2 described above, 10% ethanol, 100 mM sodium chloride and 50 mM calcium chloride, and the resulting suspension was stirred at 25°C for 48 hours. This suspension was filtered with a stainless screen having a screen-opening diameter of 32 µm, and the residue was washed with 10% ethanol containing 50 mM calcium chloride. Further, the residue was suspended in 10 liters of 10% ethanol containing 50 mM calcium chloride, and the suspension was stirred for 3 hours, and thereafter filtered with the stainless screen, and the residue was washed. Further, the residue was suspended under the same conditions, and the suspension was then stirred for 16 hours. The suspension was filtered with the stainless screen having a diameter of 32 µm, and the residue was washed.

The filtrate and the washings thus obtained were collected, and the combined mixture was subjected to ultrafiltration with an ultrafilter equipped with holofiber having an excluding molecular weight of 3000, thereby separating a filtered solution from a non-filtered solution.

This filtered solution was concentrated to a volume of about 3 liters with a rotary evaporator, and thereafter the concentrate was centrifuged, to give supernatant. This supernatant was desalted with an electric dialyzer equipped with a membrane having an excluding molecular weight of 300. To this solution was added calcium acetate so as to give a concentration of 0.1 M, and precipitates formed were removed by centrifugation. This supernatant was applied onto a DEAF-Cellulofine column A-800 column (amount of resin: 4 liters) equilibrated with 50 mM calcium acetate, and sufficiently washed with 50 mM calcium acetate and 50 mM sodium chloride. Thereafter, the elution of adsorbed substances in the column was carried out with a concentration gradient of from 50 mM to 800 mM sodium chloride. The amount collected at this time was 500 mL per fraction. The collected fraction was analyzed by cellulose acetate membrane electrophoresis [*Analytical Biochemistry*, *37,* 197-202 (1970)]. As a result, it was revealed that a sulfated saccharide which was eluted at a concentration of about 0.4 M sodium chloride (Proximity of Fraction No. 63) contained substantially no impurities.

Then, a solution of Fraction No. 63 was first concentrated to a volume of 150 mL, and thereafter sodium chloride was added so as to give a concentration of 4 M. The resulting solution was applied onto a Phenyl-Cellulofine column (amount of resin: 200 mL) (manufactured by Seikagaku Corporation) previously equilibrated with 4 M sodium chloride, and sufficiently washed with 4 M sodium chloride. Non-adsorbent sulfated saccharide fractions were collected, and desalted with an electrodialyzer equipped with a membrane having an excluding molecular weight of 300, to give 505 mL of a desalted solution.

Forty milliliters of this desalted solution was applied onto a Cellulofine GCL-90 column (4.1 cm x 87 cm) (manufactured by Seikagaku Corporation) equilibrated with 0.2 M sodium chloride containing 10% ethanol, to perform gel filtration. The collection was performed at 9.2 mL per fraction.

All of the fractions were analyzed for a total sugar content by the phenol-sulfuric acid method [*Analytical Chemistry,* **28,** 350 (1956)].

As a result, since the sulfated saccharide formed a single peak, Fraction Nos. 63 to 70, which were fractions corresponding to a central part of the peak were collected. The combined fraction was desalted with an electrodialyzer equipped with a membrane having an excluding molecular weight of 300, and thereafter lyophilized, to give 112 mg of a dried product of the compound represented by the following formula (V). The compound is referred to as 7-12SFd-F.

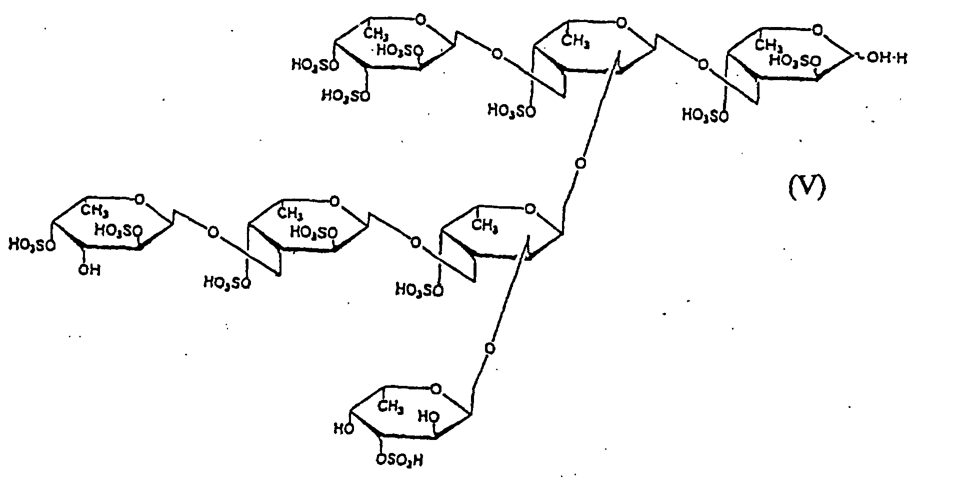

(3) Ninety-eight milligrams of F-fucoidan prepared according to the method described in item (2) of Preparation Example 1 was dissolved in 5 mL of DMSO, and 980 mg of piperidine sulfate was added thereto at room temperature. Thereafter, the resulting mixture was stirred at 80°C for 2 hours. After cooling the reaction solution, the reaction solution was dialyzed with a dialyzer having an excluding molecular weight of 1000 for 2 days. The resulting solution inside the dialyzer was applied onto a cation exchange column [Amberlite IRA-120 (Na⁺)], and thereafter the eluate was exsiccated under decompression, to give 98 mg of highly-sulfated F-fucoidan.

(4) Thirty-four milligrams of 7-12SFd-F prepared according to the method described in item (2) of Preparation Example 2 was dissolved in 4 mL of DMSO, and thereafter the same steps as those in item (3) of Preparation Example 2 were carried out, to give 34 mg of highly-sulfated 7-12SFd-F.

### Preparation Example 3

(1) Two kilograms of dried *Kjellmaniella crassifolia* was powdered with a cutter mill (manufactured by Masuko Sangyo) fitted with a screen having a hole diameter of 1 mm. After the powders were stirred in 20 liters of 80% ethanol at 25°C for 3 hours, the mixture was filtered, and the residue was washed. The resulting residue was suspended in 20 liters of a 30 mM imidazole buffer (pH 8.2) containing 50 mM calcium chloride, 100 mM sodium chloride, 10% ethanol, and 1 U of *Alteromonas* sp. SN-1009 F-fucoidan-specific degradation enzyme prepared in item (1) of Preparation Example 2. The resulting suspension was stirred at 25°C for 2 days, and thereafter filtered with a stainless screen having a hole diameter of 32 µm, and the residue was washed. The resulting residue was suspended in 40 liters of a sodium phosphate buffer (pH 6.6) containing 100 mM sodium chloride, 10% ethanol and 4 g of an alginic acid lyase (manufactured by Nagase Seikagaku Kogyo). The resulting suspension was stirred at 25°C for 4 days, and thereafter centrifuged, to give supernatant. In order to remove low-molecular weight products of alginic acid contained in the supernatant obtained, the supernatant was concentrated to a volume of 2 liters with an ultrafilter equipped with holofiber having an excluding molecular weight of 100000, and thereafter the solvent was exchanged for 100 mM sodium chloride containing 10% ethanol. To the resulting solution was added an equivolume of 400 mM calcium acetate with stirring, and thereafter the mixture was centrifuged. The pH of the resulting supernatant was adjusted to 2 with 1 N hydrochloric acid, with cooling on ice. Precipitates formed were removed by centrifugation, and the pH of the resulting supernatant was adjusted to 8 with 1 N sodium hydroxide. This solution was concentrated to a volume of 1 liter by ultrafiltration, and thereafter the solvent of the concentrate was exchanged for 100 mM sodium chloride. Precipitates formed at this time were removed by centrifugation. In order to remove hydrophobic substances in the resulting supernatant, sodium chloride was added to the supernatant so as to give a concentration of 1 M, and the resulting mixture was applied onto a 3-liter Phenyl-Cellulofine column (manufactured by Seikagaku Corporation) equilibrated with 1 M sodium chloride, to collect an effluent fraction. This fraction was concentrated with an ultrafilter, and thereafter the solvent was exchanged for 20 mM sodium chloride. The resulting solution was lyophilized, and the weight of the lyophilized product was 29.3 g.

(2) Fifteen grams of the above-mentioned lyophilized product was dissolved in 1.5 liters of 50 mM Tris-HCl buffer containing 400 mM sodium chloride and 9 U of the endo-sulfated polysaccharide-degrading enzyme (U-fucoidan-specific degradation enzyme) obtained from the culture prepared by culturing *Flavobacterium sp.* SA-0082 (FERM BP-5402) disclosed in WO97/26896. After the resulting solution was subjected to the reaction at 25°C for 6 days, the reaction mixture was concentrated to a volume of about 300 mL with an evaporator. The concentrate was placed in a dialyzing tube having an excluding molecular weight of 3500 and thoroughly dialyzed. The solution remaining in the dialysis tube was applied onto a 4-liter DEAE-Cellulofine A-800 column equilibrated with 50 mM sodium chloride, and sufficiently washed with 50 mM sodium chloride. Thereafter, the elution of adsorbed substances in the column was carried out on a concentration gradient of from 50 to 650 mM sodium chloride. Further, the elution of the adsorbed substances in the column was sufficiently carried out with 650 mM sodium chloride. Among the eluted fractions, the fractions eluted with 650 mM sodium chloride were collected as a sulfated fucogalactan fraction, and concentrated with an ultrafilter having an excluding molecular weight of 100000. Thereafter, the solvent of the concentrate was substituted with 10 mM sodium chloride, and the resulting solution was lyophilized, to give 0.85 g of a lyophilized product of sulfated fucogalactan. The sulfated fucogalactan obtained (G-fucoidan) was found to contain galactose and fucose as constituting saccharides in a molar ratio of about 2:1.

### Preparation Example 4

The fucoidan derived from *Kjellmaniella crassifolia* obtained in item (3) of Preparation Example 1 was treated with the U-fucoidan-specific degradation enzyme described in item (2) of Preparation Example 3, to prepare a degradation product.

Specifically, 16 mL of a 2.5% fucoidan solution, 12 mL of 50mM phosphate buffer (pH 7.5), 4 mL of 4M sodium chloride and a 8 mL solution of 32 mU/mL of the above-mentioned U-fucoidan-specific degradation enzyme were mixed, and the resulting mixture was reacted at 25°C for 48 hours.

The reaction solution was subjected to molecular weight fractionation by a Cellulofine GCL-300 column (manufactured by Seikagaku Corporation), and fractions of molecular weight of 2000 or less were collected. This fraction was desalted with a microacylizer G3 (manufactured by ASAHI KASEI CORPORATION), and thereafter separated into 3 fractions by a DEAE-Sepharose FF column (manufactured by Pharmacia). The fractions were desalted, and thereafter lyophilized, to give purified products in amounts of 41 mg, 69 mg and 9.6 mg, respectively. It was confirmed that these purified products have respective molecular weights of 564, 724, and 1128 according to mass spectrometry, and that these purified products are represented by the respective formulas (VI), (VII) and (VIII), as shown below, according to NMR analysis. These compounds are hereinafter referred to as 3-1S, 3-3S and 6-2SFd-U, respectively.

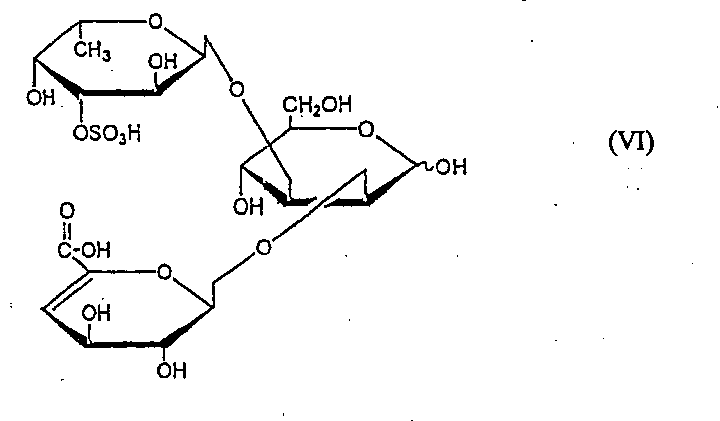

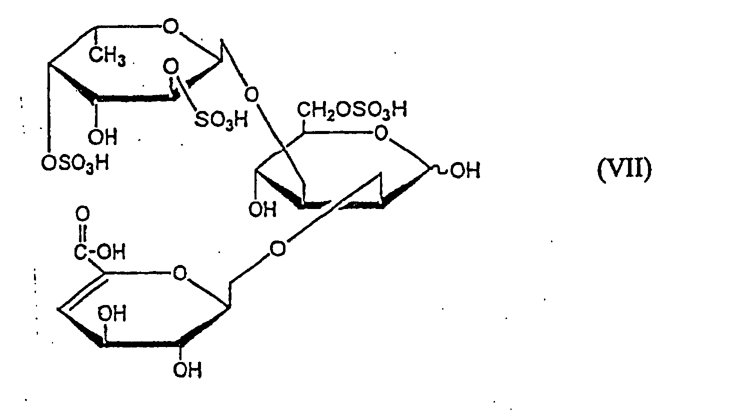

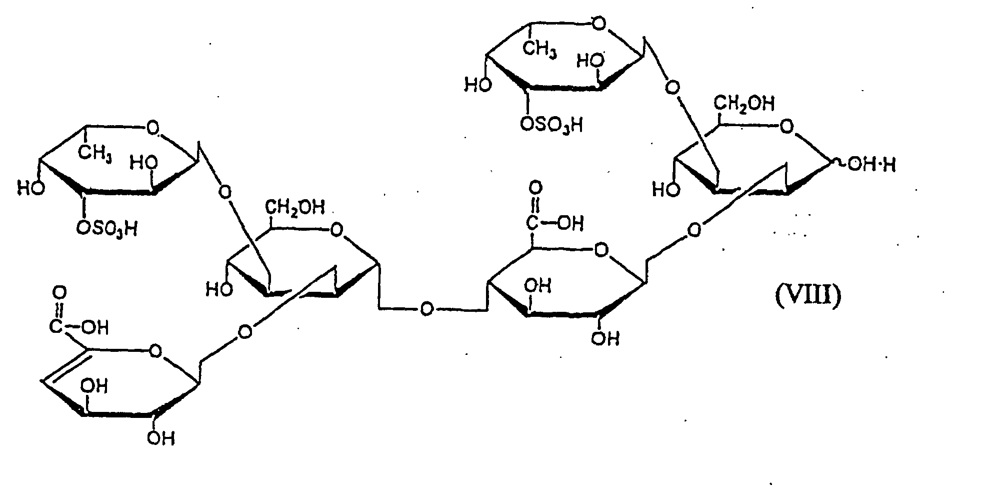

### Preparation Example 5

One kilogram of a dried product of a commercially available sporophyll of *Undaria pinnatifida* was powdered with a cutter mill fitted with a screen having a hole diameter of 1 mm. Thereafter, the powdered sporophyll was suspended in 10 liters of 80% ethanol, and the suspension was stirred for 3 hours, and thereafter filtered with a filter paper, to give a residue. The residue was suspended in 20 liters of a 40 mM sodium phosphate buffer (pH 6.5) containing 50 mM sodium chloride, and treated at 95°C for 2 hours. The treated solution was cooled to 37°C, and thereafter ethanol was added thereto so as to give a concentration of 10%. 12000 U of a commercially available alginic acid lyase K (manufactured by Nagase Seikagaku Kogyo) was added thereto, and thereafter the mixture was stirred at room temperature for 24 hours. The resulting treated solution was centrifuged, and the supernatant was concentrated to a volume of 2 liters with an ultrafilter equipped with holofiber having an excluding molecular weight of 100000. Thereafter, precipitates formed were removed by centrifugation. The resulting supernatant was cooled to 5°C, and thereafter 0.5 N hydrochloric acid was added thereto to adjust the pH to 2. Subsequently, the resulting mixture was stirred for 30 minutes, and precipitates formed were removed by centrifugation. The pH of the supernatant was adjusted to 8 with 0.5 N sodium hydroxide, and the solvent was substituted with 20 mM sodium chloride by ultrafiltration. The pH of the resulting solution was adjusted to 8, and thereafter the supernatant obtained by centrifugation was lyophilized, to give 90.5 g of a fucoidan derived from sporophyll of *Undaria pinnatifida*.

### Preparation Example 6

Two grams of the fucoidan derived from *Kjellmaniella crassifolia* prepared by the method described in item (1) of Preparation Example 1 was dissolved in 100 mL of water, and the pH of the solution was adjusted to 3 with citric acid. Thereafter, the resulting mixture was treated at 100°C for 3 hours, to give a product decomposed with the acid of the fucoidan. This product decomposed with the acid of the fucoidan was subjected to molecular weight fractionation by gel filtration with a Cellulofine GCL-300 column or a Cellulofine GCL-25 column, into fractions of a molecular weight exceeding 25000 (Fraction A), exceeding 10000 to 25000 (Fraction B) and exceeding 5000 to 10000 (Fraction C), exceeding 2000 to 5000 (Fraction D), exceeding 500 to 2000 (Fraction E) and 500 or less (Fraction F). Further, each of these fractions and the product decomposed with the acid were desalted, and then lyophilized, to give each fraction of the product degraded with the acid, and the product degraded with the acid.

### Preparation Example 7

Five kilograms of a commercially available, salt-preserved *Nemacystus decipiens* was mixed with 20 liters of ethanol, and the salt-preserved *Nemacystus decipiens* in ethanol was cut into thin pieces with scissors,. The resulting mixture was allowed to stand overnight, and then filtered with a filter paper. The resulting residue was suspended in 12.5 liters of water, and the suspension was allowed to stand at 95°C for 2 hours. After the treated solution was filtered with a filter paper, 2600 mL of a 2.5% cetyl pyridinium chloride solution containing 350 mM sodium chloride was added thereto, and the resulting mixture was allowed to stand for 3 days. The supernatant portion was discarded, the precipitate portion was centrifuged, and the supernatant was also discarded. To the precipitates obtained was added 2.5 liters of 350 mM sodium chloride, and thereafter the mixture was homogenized with a homogenizer and centrifuged. The washing steps were repeated 3 times. Four-hundred milliliters of 400 mM sodium chloride was added to the precipitates obtained. Thereafter, the mixture was homogenized with a homogenizer, and ethanol was added thereto so as to give a concentration of 80%. The mixture was stirred for 30 minutes, and then filtered with a filter paper. Five hundred milliliters of 80% ethanol saturated with sodium chloride was added to the residue obtained, and thereafter the mixture was homogenized with a homogenizer. Ethanol saturated with sodium chloride was added to make up a volume of 1 liter, and the mixture was stirred for 30 minutes and then filtered with a filter paper. The washing steps were repeated until the absorbance at 260 nm of the filtrate became substantially 0 (zero) (usually 5 times). The residue obtained was dissolved in 1.5 liters of 2 M sodium chloride, and thereafter insoluble substances were removed by centrifugation. The resulting solution was allowed to flow through a column containing 100 mL of DEAE-Cellulofine A-800 equilibrated with 2 M sodium chloride. Effluent fractions were concentrated to a volume of 2 liters with an ultrafilter equipped with holofiber having an excluding molecular weight of 100000, and thereafter the solvent was substituted with 2 mM sodium chloride by an ultrafilter. This solution was centrifuged, and the resulting supernatant was lyophilized, to give 22.9 g of a fucoidan derived from *Nemacystus decipiens*.

### Preparation Example 8

Five kilograms of *stichopus* were dissected, and the organs were removed to collect somatic layers. Five-hundred milliliters of acetone was added per 200 g of the wet weight of the somatic layers, and the mixture was treated with a homogenizer. Thereafter, the homogenate was filtered, and the residue was washed with acetone until no more colored substances remained. This residue was dried with suction, to give 140 g of a dried product. To this dried product was added 2.8 liters of a 0.4 M aqueous sodium chloride, and the mixture was treated at 100°C for 1 hour. Thereafter, the mixture was filtered, and the residue was sufficiently washed with a 0.4 M aqueous sodium chloride, to give 3.7 liters of an extract. To this extract was added 5% cetyl pyridinium chloride until no more precipitates were formed, and the formed precipitates were harvested by centrifugation. The precipitates were suspended in a 0.4 M aqueous sodium chloride, and again centrifuged. One liter of a 4 M aqueous sodium chloride was added to the resulting precipitates, and the mixture was treated with a homogenizer. Thereafter, 4 liters of ethanol was added thereto with stirring, and the resulting mixture was stirred for 1 hour, and thereafter filtered, to give precipitates. The steps of suspending the precipitates in 80% ethanol and thereafter filtering the suspension were repeated until the absorbance at 260 nm of the supernatant became substantially zero (0). The precipitates obtained were suspended in 2 liters of a 2 M aqueous sodium chloride, and insoluble substances were removed by centrifugation. The supernatant was ultrafiltered with an ultrafilter equipped with a membrane having an excluding molecular weight of 30000, and completely desalted. Thereafter, the resulting product was lyophilized, to give 3.7 g of a fucoidan derived from sea cucumbers.

### Preparation Example 9

Six-hundred and twenty-five grams of a commercially available, salt-preserved *Cladosiphon okamuranus* was suspended in 4375 mL of a 30 mM sodium phosphate buffer (pH 6.0), and the suspension was treated with a homogenizer at 8000 rotations per minute for 5 minutes. Thereafter, the homogenate was treated at 95°C for 1 hour, and centrifuged, to give supernatant. Ten grams of activated carbon was added to this supernatant. Thereafter, the resulting mixture was stirred for 30 minutes, and centrifuged, to give supernatant. The resulting supernatant was concentrated to a volume of 2 liters with an ultrafilter equipped with holofiber having an excluding molecular weight of 100000. Thereafter, the solvent was substituted with a 20 mM sodium chloride, and the resulting solution was lyophilized, to give 10.9 g of a dried product of a fucoidan fraction derived from *Cladosiphon okamuranus*.

### Preparation Example 10

One kilogram of a dried product of powdered *Fucus vesiculosus* was suspended in 10 liters of 80% ethanol, and the suspension was stirred for 3 hours, and thereafter filtered with a filter paper, to give a residue. The residue was suspended in 30 liters of a 30 mM phosphate buffer (pH 6) containing 100 mM sodium chloride, and treated at 95°C for 2 hours. After the treated solution was cooled to 37°C, 100 g of activated carbon was added thereto, and the mixture was stirred for 30 minutes. After 3000 U of a commercially available alginic acid lyase K was added thereto, ethanol was added so as to give a concentration of 10%, and the resulting mixture was stirred at room temperature for 24 hours. The resulting treated solution was centrifuged, and the supernatant was concentrated to a volume of 2 liters with an ultrafilter equipped with holofiber having an excluding molecular weight of 100000. Thereafter, precipitates formed were removed by centrifugation, and the supernatant was ultrafiltered with adding 100 mM sodium chloride, to remove a pigment. The non-filtered solution obtained was cooled to 5°C, and thereafter 0.5 N hydrochloric acid was added thereto to adjust the pH to 2. Subsequently, the resulting solution was stirred for 30 minutes, and precipitates formed were removed by centrifugation. The pH of the supernatant was adjusted to 8 with 0.5 N sodium hydroxide, and the solvent was substituted with 20 mM sodium chloride by ultrafiltration. The pH of the resulting solution was adjusted to 8, and thereafter the supernatant obtained after centrifugation was lyophilized, to give 71 g of a fucoidan derived from *Fucus vesiculosus*.

### Example 1

MG-63 cells (human osteosarcoma cell line, ATCC CRL-1427), human fibroblasts (hFBs), were cultured in a DMEM medium (manufactured by Bio Whittaker) containing 10% fetal calf serum (FCS, manufactured by Bio Whittaker) in the presence of 5% CO₂ at 37°C until the cells became confluent. The cells were suspended in the above-mentioned medium using Reagent Pack™ (manufactured by Bio Whittaker) so as to have a concentration of 5 x 10⁴ cells/mL. The cell suspension was added to each well of a 48-well microtiter plate in a volume of 500 µL each. After 2 to 3 day of culture, the medium was discarded at a point where the hFB monolayer became subconfluent. To the cells was added a 1.5% FCS-DMEM medium containing a solution prepared by dissolving each of various kinds of fucoidans (the fucoidan derived from *Kjellmaniella crassifolia* described in item (1) of Preparation Example 1, the fucoidan derived from *Kjellmaniella crassifolia* described in item (3) of Preparation Example 1, the U-fucoidan prepared according to the method described in item (2) of Preparation Example 1, the fucoidan derived from sporophyll of *Undaria pinnatifida* described in Preparation Example 5, or the fucoidan derived from *Fucus vesiculosus* described in Preparation Example 10) as a test substance in physiological saline. To the cells was added a 1.5% FCS-DMEM medium containing the same volume of physiological saline alone as the control. The cells were cultured for 24 hours or 48 hours, and thereafter the amount of TGF-β₁ in the culture supernatant was assayed by ELISA kit (TGF-β₁ Emax™ Immuno Assay System, manufactured by Promega), and the enhancing action for TGF-β₁ production was assayed for each of the test samples. The results are shown in Table 1. Also, the enhancing action for TGF-β₁ production was assayed for each of 3-1S and 3-3S described in Preparation Example 4, 7-12SFd-F described in item (2) of Preparation Example 2 and the highly-sulfated 7-12SFd-F described in item (4) of Preparation Example 2 in the same manner. The results are shown in Table 2. The concentrations of the respective test samples in the media are shown in Tables 1 and 2. In addition, the influence of the various kinds of fucoidans and the degradation products thereof used in this Example on MG-63 cells was examined by using MTT assay. As a result, toxicity to MG-63 cells was not found.

**Table 1**

| Various Kinds of Fucoidans (µg/mL) | Amount of TGF-β₁ (pg/mL) | |
|---|---|---|
| | 24 Hours | 48 Hours |
| Control | N.D. | 475 |

| Fucoidan Derived from *Kjellmaniella Crassifolia* Described in Item (1) of Preparation Example 1 | | |
|---|---|---|
| 1 | 60 | 703 |
| 10 | N.T. | 562 |

| Fucoidan Derived from *Kjellmaniella Crassifolia* Described in Item (3) of Preparation Example 1 | | |
|---|---|---|
| 1 | 558 | 790 |
| 10 | 320 | 858 |
| 100 | 461 | N.T. |

| U-Fucoidan | | |
|---|---|---|
| 1 | 211 | 757 |
| 10 | 154 | 764 |
| 100 | 1084 | 1010 |

| Fucoidan Derived from Sporophyll of *Undaria Pinnatifida* | | |
|---|---|---|
| 1 | 643 | 1272 |
| 10 | 989 | 1059 |

| Fucoidan Derived from *Fucus Vesiculosus* | | |
|---|---|---|
| 1 | 985 | 1523 |
| 10 | 454 | 743 |
| N.D. means below the detection limit, and | | |
| N.T. means not tested. | | |

**Table 2**

| Degradation Products of Various Kinds of Fucoidans (µg/mL) | Amount of TGF-β₁ (pg/mL) | |
|---|---|---|
| | 24 Hours | 48 Hours |
| Control | 305.5 | 409.2 |

| 3-1S | | |
|---|---|---|
| 1 | 309.6 | 567.8 |
| 10 | 431.1 | 607.4 |
| 100 | 769.86 | 484.4 |

| 3-3S | | |
|---|---|---|
| 1 | 321.9 | 518.4 |
| 10 | 504.9 | 610.0 |

| 7-12SFd-F | | |
|---|---|---|
| 1 | 372.3 | 614.1 |

| Highly Sulfated 7-12SFd-F | | |
|---|---|---|
| 1 | 319.0 | 502.1 |
| 10 | 375.2 | 463.8 |

As shown in Tables 1 and 2, there was confirmed the enhancing actions for TGF-β₁ production by various kinds of fucoidans and the degradation products thereof in human fibroblasts MG-63.

### Example 2

The enhancement of TGF-β₁ production by fucoidan in HT-1080 cells was assayed in the same manner as in Example 1 using HT-1080 cells (human fibrosarcoma cell line, ATCC CRL-121), human fibroblasts (hFBs), in place of MG-63 cells of Example 1. A solution prepared by dissolving each of various kinds of fucoidans (the fucoidan derived from *Kjellmaniella crassifolia* described in item (1) of Preparation Example 1, the fucoidan derived from *Kjellmaniella crassifolia* described in item (3) of Preparation Example 1, the U-fucoidan prepared according to the method described in item (2) of Preparation Example 1, the F-fucoidan prepared according to the method described in item (2) of Preparation Example 1, the fucoidan derived from sporophyll of *Undaria pinnatifida* described in Preparation Example 5, or the fucoidan derived from *Fucus vesiculosus* described in Preparation Example 10) in physiological saline was used as a test sample. Also, the same volume of physiological saline was used as the control. The results are shown in Tables 3 and 4. Also, the enhancing action for TGF-β₁ production was assayed for each of 3-1S and 3-3S described in Preparation Example 4, 7-12SFd-F described in item (2) of Preparation Example 2 and the highly-sulfated F-fucoidan described in item (3) of Preparation Example 2 in the same manner. The results are shown in Table 5. In addition, the influence of the various kinds of fucoidans and the degradation products thereof used in this Example on HT-1080 was examined using the MTT assay. As a result, toxicity to HT-1080 cells was not found.

**Table 3**

| Various Kinds of Fucoidans (µg/mL) | Amount of TGF-β₁ (pg/mL) | |
|---|---|---|
| | 24 Hours | 48 Hours |
| Control | 1073 | 2807 |

| Fucoidan Derived from *Kjellmaniella Crassifolia* Described in Item (1) of Preparation Example 1 | | |
|---|---|---|
| 1 | 1496 | 4208 |
| 10 | 1253 | N.T. |

| Fucoidan Derived from *Kjellmaniella Crassifolia* Described in Item (3) of Preparation Example 1 | | |
|---|---|---|
| 1 | 5543 | N.T. |
| 10 | 3767 | N.T. |
| 100 | 2954 | N.T. |
| N.T. means not tested. | | |

**Table 4**

| Various Kinds of Fucoidans (µg/mL) | Amount of TGF-β₁ (pg/mL) | |
|---|---|---|
| | 24 Hours | 48 Hours |
| Control | 1073 | 2807 |

| F-Fucoidan | | |
|---|---|---|
| 1 | 1655 | 4447 |
| 10 | 1605 | 3632 |

| U-Fucoidan | | |
|---|---|---|
| 1 | 2108 | 5057 |
| 10 | 2008 | 5594 |
| 100 | 2140 | 5538 |

| Fucoidan Derived from Sporophyll of *Undaria Pinnatifida* | | |
|---|---|---|
| 1 | 1944 | 5702 |
| 10 | 2052 | 4236 |

| Fucoidan Derived from *Fucus Vesiculosus* | | |
|---|---|---|
| 1 | 1832 | 5859 |

**Table 5**

| Degradation Products of Various Kinds of Fucoidans (µg/mL) | Amount of TGF-β₁ (pg/mL) | |
|---|---|---|
| | 24 Hours | 48 Hours |
| Control | 4687 | 4358 |

| 3-1S | | |
|---|---|---|
| 1 | 7112 | 4820 |

| 3-3S | | |
|---|---|---|
| 100 | 5086 | 5655 |

| 7-12SFd-F | | |
|---|---|---|
| 1 | 5145 | 5485 |
| 100 | 5495 | 6851 |

| Highly Sulfated F-Fucoidan | | |
|---|---|---|
| 100 | 5428 | 6750 |

As shown in Tables 3 to 5, there was confirmed the enhancing actions for TGF-β₁ production by various kinds of fucoidans and the degradation products thereof in human fibroblasts HT-1080.

### Example 3

The enhancement of TGF-β₁ production by fucoidan was assayed in HT-1080 in the same manner as in Example 1 using HT-1080 cells (human fibrosarcoma cell line, ATCC CRL-121), human fibroblasts (hFBs), in place of MG-63 cells of Example 1. A solution prepared by dissolving each of various kinds of fucoidans (the F-fucoidan-containing filtrate described in item (4) of Preparation Example 1 or the F-rich fucoidan described in item (5) of Preparation Example 1) in physiological saline was used as a test sample. The same volume of physiological saline was used as the control. The results are shown in Table 6. In addition, the influence of the various kinds of fucoidans used in this Example on cell proliferation was examined using a WST-1 assay. As a result, toxicity to HT-1080 cells was not found.

**Table 6**

| Various Kinds of Fucoidans | Amount of TGF-β₁ (pg/mL) | |
|---|---|---|
| | 24 Hours | 48 Hours |
| Control | 924 | 2337 |

| F-Fucoidan-Containing Filtrate [%(v/v)] | | |
|---|---|---|
| 1 | 1646 | 3165 |
| 3 | 978 | 2796 |

| F-rich-Fucoidan (µg/mL) | | |
|---|---|---|
| 0.1 | 1118 | N.T. |
| 1 | 1183 | 2877 |
| 10 | 1109 | N.T. |
| N.T. means not tested. | | |

As shown in Table 6, there was confirmed the enhancing action for TGF-β₁ production by the F-fucoidan-containing filtrate or the F-rich fucoidan in human fibroblasts HT-1080 cells.

### Example 4

The enhancing action for TGF-β₁ production by fucoidan in HT-1080 cells was assayed in the same manner as in Example 2. A solution prepared by dissolving each of various kinds of fucoidans (the fucoidan derived from *Laminaria japonica,* the fucoidan derived from *Lessonia nigrescence,* the fucoidan derived from *Ecklonia maxima,* the fucoidan derived from *Durvilliae*, the fucoidan derived from *Ascophyllum nodosum*, or the fucoidan derived from *Cladosiphon okamuranus*, each prepared in item (3) of Preparation Example 1) in physiological saline was used as a test sample. The same volume of physiological saline was used as the control. The results are shown in Tables 7 and 8. In addition, the influence of the various kinds of fucoidans used in this Example on cell proliferation was examined using a WST assay. As a result, toxicity to HT-1080 cells was not found.

**Table 7**

| Various Kinds of Fucoidans (µg/mL) | Amount of TGF-β₁ (pg/mL) |
|---|---|
| Control | 975.5 |

| Fucoidan Derived from *Laminaria Japonica* | |
|---|---|
| 1 | 1210.2 |
| 10 | 1776.3 |
| 100 | 1253.5 |

| Fucoidan Derived from *Lessonia* | |
|---|---|
| 0.1 | 995.1 |
| 1 | 2633.9 |
| 10 | 2360.7 |

| Fucoidan Derived from *Ecklonia* | |
|---|---|
| 0.1 | 1054.1 |
| 1 | 2965.5 |
| 10 | 3687.4 |

**Table 8**

| Various Kinds of Fucoidans (µg/mL) | Amount of TGF-β₁ (pg/mL) |
|---|---|
| Control | 975.5 |

| Fucoidan Derived from *Durvilliae* | |
|---|---|
| 0.1 | 1223.9 |
| 1 | 1666.2 |
| 10 | 1692.6 |
| 100 | 1190.7 |

| Fucoidan Derived from *Ascophyllum* | |
|---|---|
| 1 | 1151.7 |
| 10 | 1805.6 |

| Fucoidan Derived from *Cladosiphon Okamuranus* | |
|---|---|
| 0.1 | 3997.5 |
| 1 | 4670.3 |
| 10 | 4670.1 |
| 100 | 1054.1 |

As shown in Tables 7 and 8, there was confirmed the enhancing actions for TGF-β₁ production by various kinds of fucoidans in human fibroblasts HT-1080.

### Example 5

MG-63 cells (human osteosarcoma cell line, ATCC CRL-1427), human fibroblasts (hFBs), were cultured in a DMEM medium (manufactured by Bio Whittaker) containing 10% fetal calf serum (FCS, manufactured by Bio Whittaker) in the presence of 5% CO₂ at 37°C until the cells became confluent. The cells were suspended in the above-mentioned medium using Reagent Pack™ (manufactured by Bio Whittaker) so as to have a concentration of 5 x 10⁴ cells/mL. The cell suspension was added to each well of a 48-well microtiter plate in a volume of 500 µl each. After 2 to 3 day of culture, the medium was discarded at a point where the hFB monolayer became subconfluent. In an *in vitro* type I pro-collagen production system, to the cells was added a 1.5% FCS-DMEM medium containing a solution prepared by dissolving the F-fucoidan-containing filtrate prepared in item (4) of Preparation Example 1 or the F-rich fucoidan described in item (5) of Preparation Example 1 in physiological saline as a test sample. To the cells was added a 1.5% FCS-DMEM medium containing the same volume of physiological saline alone as the control. The cells were cultured for 24 hours or 48 hours, and thereafter the amount of type I pro-collagen in the culture supernatant was assayed using a Pro-collagen type I C-peptide [PIP] EIA Kit (manufactured by Takara Shuzo Co., Ltd.). The results are shown in Table 9. In addition, the influence of the various kinds of fucoidans used in this Example on cell proliferation was examined using a WST-1 assay. As a result, toxicity to MG-63 cells was not found.

**Table 9**

| Various Kinds of Fucoidans | Amount of Type-I Pro-collagen (ng/ml) | |
|---|---|---|
| | 24 Hours | 48 Hours |
| Control | 312 | 387 |

| F-Fucoidan-Containing Filtrate [%(v/v)] | | |
|---|---|---|
| 0.3 | N.T. | 503 |
| 1 | 475 | 654 |
| 3 | 421 | 519 |
| 10 | 389 | 465 |

| F-rich-Fucoidan (µg/ml) | | |
|---|---|---|
| 0.1 | 389 | 397 |
| 1 | 496 | 564 |
| 10 | 487 | 564 |
| 100 | 356 | 414 |
| N.T. means not tested. | | |

As shown in Table 9, there was confirmed the enhancing actions for type I pro-collagen production by the F-fucoidan-containing filtrate and the F-rich fucoidan in human fibroblasts MG-63.

### Example 6

The influence on amount of type I pro-collagen produced was examined in the same manner as in Example 5 for each of the fucoidan derived from *Kjellmaniella crassifolia*, the fucoidan derived from *Laminaria japonica*, the fucoidan derived from *Lessonia nigrescence*, the fucoidan derived from *Ecklonia maxima*, and the fucoidan derived from *Durvilliae*, each prepared in item (3) of Preparation Example 1 as a test sample. The results are shown in Table 10. Incidentally, the influence of the various kinds of fucoidans used in this Example on cell proliferation was examined. As a result, toxicity to MG-63 cells was not found.

**Table 10**

| Various Kinds of Fucoidans (µg/ml) | Amount of type-I Pro-collagen (ng/ml) |
|---|---|
| | 24 Hours |
| Control | 387 |

| Fucoidan Derived from *Kjellmaniella Crassifolia* | |
|---|---|
| 0.1 | 409 |
| 1 | 549 |
| 10 | 502 |
| 100 | 395 |

| Fucoidan Derived from *Laminaria Japonica* | |
|---|---|
| 1 | 379 |
| 10 | 432 |
| 100 | 347 |

| Fucoidan Derived from *Lessonia* | |
|---|---|
| 0.1 | 351 |
| 1 | 387 |

| Fucoidan Derived from *Ecklonia* | |
|---|---|
| 0.1 | 340 |
| 1 | 411 |
| 10 | 386 |

| Fucoidan Derived from *Durvilliae* | |
|---|---|
| 1 | 355 |
| 10 | 360 |

As shown in Table 10, there was confirmed the enhancing actions for type I pro-collagen production by fucoidans derived from various kinds of algae in human fibroblasts MG-63.

### Example 7

Each of Hs-68 cells (normal human newborn foreskin cell line, ATCC CRL-1635), which are human skin fibroblasts (hSFBs), MG-63 cells (human osteosarcoma cell line, ATCC CRL-1427), which are human fibroblasts (hFBs), or HT-1080 cells (human fibrosarcoma cell line, ATCC CRL-121) was cultured in a DMEM medium (manufactured by Bio Whittaker) containing 10% fetal calf serum (FCS, manufactured by Bio Whittaker) in the presence of 5% CO₂ at 37°C until the cells became confluent. The cells were suspended in the above-mentioned medium using Reagent Pack™ (manufactured by Bio Whittaker) so as to have a concentration of 5 x 10⁴ cells/mL. The cell suspension was added to each culture flask with a diameter of 100 mm (manufactured by IWAKI) in a volume of 10 mL each. After 2 to 3 day of culture, the medium was discarded at a point where the hSFB monolayer or the hFB monolayer became subconfluent. To the cells was added a 1.5% FCS-DMEM medium containing a solution prepared by dissolving each of various kinds of fucoidans (the fucoidan derived from *Kjellmaniella crassifolia* described in item (1) of Preparation Example 1, the fucoidan derived from *Kjellmaniella crassifolia* described in item (3) of Preparation Example 1, the F-fucoidan prepared according to the method described in item (2) of Preparation Example 1, the U-fucoidan prepared according to the method described in item (2) of Preparation Example 1, or the fucoidan derived from sporophyll of *Undaria pinnatifida* described in Preparation Example 5) in physiological saline as a test sample. Also, to the cells was added a 1.5% FCS-DMEM medium containing the same volume of physiological saline alone as the control. The cells were cultured for 24 hours, and thereafter the culture supernatant was discarded. The cells were washed with PBS solution, and thereafter released from the flask with a 5.3 mM EDTA-PBS solution (manufactured by GIBCO/BRL). Next, each of integrin α1β1 and integrin α2β1 on the surface of the fibroblasts was subjected to FITC staining with anti-human integrin antibodies (α1β1/CD49a/CD29, manufactured by ENDOGEN, α2β1/CD49b/CD29, manufactured by CHEMICON), and the expression levels of each integrin were determined with FACScan (manufactured by Ortho Cytoron Omron). The results are shown in Tables 11 and 12. The experiment was carried out twice, and an average value was taken. The values in the tables were calculated provided that the value for the control was defined as 100%.

**Table 11**

| Various Kinds of Fucoidans (µg/mL) | Expression Level of Integrin α1β1 (%) | | |
|---|---|---|---|
| | Hs-68 | HT-1080 | MG-63 |
| Control | 100 | 100 | 100 |

| Fucoidan Derived from *Kjellmaniella Crassifolia* Described in Item (1) of Preparation Example 1 | | | |
|---|---|---|---|
| 1 | 86.5 | 95.0 | 84.9 |
| 10 | 94.0 | 97.3 | 76.7 |

| Fucoidan Derived from *Kjellmaniella Crassifolia* Described in Item (3) of Preparation Example 1 | | | |
|---|---|---|---|
| 1 | 85.2 | 95.6 | 87.6 |
| 10 | 87.6 | 96.4 | 80.6 |

| F-Fucoidan | | | |
|---|---|---|---|
| 1 | 90.8 | 99.3 | 84.3 |
| 10 | 95.6 | 96.5 | 77.7 |

| U-Fucoidan | | | |
|---|---|---|---|
| 1 | 90.9 | 93.3 | 111.3 |
| 10 | 88.6 | 96.1 | 94.0 |

| Fucoidan Derived from Sporophyll of *Undaria Pinnatifida* | | | |
|---|---|---|---|
| 1 | N.D. | 96.6 | 69.8 |
| 10 | N.D. | 95.1 | 69.5 |
| N.D. means below the detection limit. | | | |

**Table 12**

| Various Kinds of Fucoidans (µg/mL) | Expression Level of Integrin α2β1 (%) | | |
|---|---|---|---|
| | Hs-68 | HT-1080 | MG-63 |
| Control | 100 | 100 | 100 |

| Fucoidan Derived from *Kjellmaniella Crassifolia* Described in Item (1) of Preparation Example 1 | | | |
|---|---|---|---|
| 1 | 92.7 | 97.1 | 97.6 |
| 10 | 100.9 | 101.8 | 98.5 |

| Fucoidan Derived from *Kjellmaniella Crassifolia* Described in Item (3) of Preparation Example 1 | | | |
|---|---|---|---|
| 1 | 92.3 | 94.4 | 92.5 |
| 10 | 97.0 | 101.8 | 90.6 |

| F-Fucoidan | | | |
|---|---|---|---|
| 1 | 96.4 | 99.4 | 108.7 |
| 10 | 100.9 | 99.9 | 98.0 |

| U-Fucoidan | | | |
|---|---|---|---|
| 1 | 101.3 | 95.1 | 111.5 |
| 10 | 107.1 | 97.6 | 93.2 |

| Fucoidan Derived from Sporophyll of *Undaria Pinnatifida* | | | |
|---|---|---|---|
| 1 | N.D. | 103.4 | 76.2 |
| 10 | N.D. | 95.0 | 76.0 |
| N.D. means below the detection limit. | | | |

As shown in Tables 11 and 12, the expression levels of integrins α1β1 and α2β1 on the cell surface of the human fibroblasts were slightly decreased by addition of fucoidan, as compared to those of the control in most cases. In other words, almost no increase in expression of integrins α1β1 and α2β1 was observed by the fucoidan. It can be seen from this finding that the effects of the fucoidans in the present invention are not connected with increase in the expression of integrins α1β1 and α2β1.

### Reference Example 1

Female HOS:HR-1 hairless mice were purchased from Nippon SLE, and used for an experiment from 5 week-old after preliminary rearing. Wrinkle formation was induced by irradiating the backside skin with a low level (a level which would not cause erythema) of ultraviolet (UVB 290 to 320 nm), to generate mouse skin wrinkle-forming models. Specifically, the UVB irradiation level was 65 mJ/cm², 5 times a week, on the first week, the UVB irradiation level being increased by 10 mJ/cm² every week thereon, and the UVB irradiation level was 95 mJ/cm² from the fourth week to the twelfth week. As the control group, normal mice of the same week-old without UVB irradiation were used. Using the mice which were on the twelfth week of UVB irradiation, the irradiated skin was observed with the passage of time, and changes in wrinkle formation were evaluated by numerical expression according to the Bisett method (Bisett DL, et al.: *Photochem & Photobiol,* **46:** 367, 1987). Specifically, the evaluations of wrinkle were: 0: no coarse wrinkle; 1: a few shallow coarse wrinkles; 2: some coarse wrinkles; and 3: several deep coarse wrinkles. In addition, skin elasticity was determined using a Cutometer SEM 575 (manufactured by Integral). The mice were reared without UVB irradiation from the thirteenth week to the twenty-fourth week, and skin on the tested site was taken, to prepare specimens of tissues. Thereafter, the thickness of skin was determined using hematoxylineosin staining, and compared with that of the control group. Also, regarding the amount of collagen in skin, skin was harvested, and the collagen content in 1 mg of total protein was determined using a Sircol Collagen Assay kit (manufactured by Biocolor). The results are shown in Tables 13 to 16. The numerical figures in the tables show an average value ± standard error of 5 cases.

On the second week of UVB irradiation, the skin of the mice of the group with UBV irradiation lost shininess, which had been originally found in normal skin and became dried, as compared to that of the control group. Little wrinkle was observed on the fourth week of UBV irradiation, intermediate wrinkle was observed on the eighth week, and large wrinkle was observed on the eleventh week and weeks subsequent thereafter. Also, the skin elasticity was obviously lowered in the group with UVB irradiation. The thickness of the skin was obviously increased. The amount of collagen in skin was obviously lowered. Incidentally, the numerical figures in the tables show an average value ± standard error of 5 cases.

**Table 13**

| Influence of Chronic Low Ultraviolet Irradiation Level on Wrinkle Formation on Backside Skin of Mouse | |
|---|---|
| Mouse Group | Evaluation of Wrinkle 12th Week |
| Group with UVB Irradiation (n=5) | 3 ± 0.25 |
| Normal Control Group (n=5) | 0 |

**Table 14**

| Influence of Chronic Low Ultraviolet Irradiation Level on Skin Elasticity on Backside of Mouse | |
|---|---|
| Mouse Group | Skin Elasticity |
| | 12th Week |
| Group with UVB Irradiation (n=5) | 0.641 ± 0.065 |
| Normal Control Group (n=5) | 0.752 ± 0.073 |
| The value for the theoretical maximum elasticity is 1. | |

**Table 15**

| Changes in Thickness of Skin by Chronic Low Ultraviolet Irradiation Level on Backside of Mouse | |
|---|---|
| Mouse Group | Thickness of Skin (µm) |
| | 24th Week |
| Group with UVB Irradiation (n=5) | 106 ± 6.607 |
| Normal Control Group (n=5) | 21 ± 1.909 |
| The values for thickness of skin in the table indicates the distance between stratum corneum epidermis and stratum basale epidermis in the backside. | |

**Table 16**

| Influence of Chronic Low Ultraviolet Irradiation Level on Amount of Collagen in Skin on Backside of Mouse | |
|---|---|
| Mouse Group | Amount of Collagen in Skin (µg/mg) |
| | 24th Week |
| Group with UVB Irradiation (n=5) | 26 ± 2.513 |
| Normal Control Group (n=5) | 42 ± 1.475 |

### Example 8

Female HOS:HR-1 hairless mice were purchased from Nippon SLC, and used for an experiment from 5 week-old after preliminary rearing. The process and the conditions for generating mouse skin wrinkle-forming models were the same as those for the experimental method described in Reference Example 1. The F-fucoidan-containing filtrate prepared in item (4) of Preparation Example 1 was previously applied onto the backside of the mice in an amount of 250 µl per mouse from the first week during the period in which wrinkle was induced by UBV irradiation. 10% Ethanol containing 1 mM potassium chloride was applied in the same manner to the group applied with the solvent. The administration was once a day, 5 times a week, for 12 consecutive weeks. The observation of changes in wrinkle and the determination of the skin elasticity were carried out in the same manner as in Reference Example 1. Further, from the thirteenth week and subsequent weeks thereafter, the F-fucoidan-containing filtrate prepared in item (4) of Preparation Example 1 was applied for additional 12 consecutive weeks without UVB irradiation. The observation of changes in wrinkle, and the determinations of the skin elasticity, the thickness of skin and the amount of collagen in skin were carried out in the same manner as in Reference Example 1. The results are shown in Tables 17 to 20. The numerical figures in the tables show an average value ± standard error of 5 cases. As a result, the group applied with the F-fucoidan-containing filtrate significantly prevented the formation of wrinkle and also prevented the loss of the skin elasticity, as compared to the group applied with the solvent and the group with UVB irradiation. In addition, the group applied with the F-fucoidan-containing filtrate prevented an increase in thickness of skin by UVB. The group applied with the F-fucoidan-containing filtrate prevented a decrease in amount of collagen in skin by UVB.

**Table 17**

| Prophylactic Action of Wrinkle Formation on Backside Skin of Mouse by Continuous Application of F-Fucoidan-Containing Filtrate | | |
|---|---|---|
| Mouse Group | Evaluation of Wrinkle | |
| | 12th Week | 24th Week |
| Group Applied with F-Fucoidan-containing Filtrate (n=5) | 2.15 ± 0.205 | 1.5 ± 0.187 |
| Group Applied with Solvent (n=5) | 3 ± 0.25 | 2.8 ± 0.215 |
| Group with UVB Irradiation (n=5) | 3 ± 0.25 | 2.85 ± 0.225 |
| Normal Control Group (n=5) | 0 | 0 |

**Table 18**

| Prophylactic Action of Lowering of Skin Elasticity on Backside of Mouse by Continuous Application of F-Fucoidan-Containing Filtrate | | |
|---|---|---|
| | Skin Elasticity | |
| Mouse Group | 12th Week | 24th Week |
| Group Applied with F-Fucoidan-Containing Filtrate (n=5) | 0.698 ± 0.075 | 0.739 ± 0.065 |
| Group Applied with Solvent (n=5) | 0.609 ± 0.065 | 0.571 ± 0.053 |
| Group with UVB Irradiation (n=5) | 0.641 ± 0.065 | 0.567 ± 0.055 |
| Normal Control Group (n=5) | 0.752 ± 0.073 | 0.762 ± 0.067 |
| The value for the theoretical maximum elasticity is 1. | | |

**Table 19**

| Changes in Thickness of Skin of Backside of Mouse by Continuous Application of F-Fucoidan-Containing Filtrate | |
|---|---|
| Mouse Group | Thickness of Skin (µm) 24th Week |
| Group Applied with F-Fucoidan-Containing Filtrate (n=5) | 43 ± 3.448 |
| Group Applied with Solvent (n=5) | 96 ± 6.333 |
| Group with UVB Irradiation (n=5) | 106 ± 6.606 |
| Normal Control Group (n=5) | 21 ± 1.909 |
| The values for thickness of skin in the table indicate the distance between stratum corneum epidermis and stratum basale epidermis in the backside. | |

**Table 20**

| Changes in Amount of Collagen in Skin of Mouse by Continuous Application of F-Fucoidan-Containing Filtrate | |
|---|---|
| Mouse Group | Amount of Collagen in Skin (µg/mg) |
| | 24th Week |
| Group Applied with F-Fucoidan-Containing Filtrate (n=5) | 39 ± 1.758 |
| Group Applied with Solvent (n=5) | 30 ± 2.712 |
| Group with UVB Irradiation (n=5) | 26 ± 2.513 |
| Normal Control Group (ri=5) | 42 ± 1.475 |

### Example 9

Female HOS:HR-1 hairless mice were purchased from Nippon SLC, and used for an experiment from 5 week-old after preliminary rearing. Wrinkle was induced in the same manner as in Reference Example 1, and thereafter the observation of changes in wrinkle and the determination of the skin elasticity were carried out in the same manner as in Reference Example 1. The F-fucoidan-containing filtrate prepared in item (4) of Preparation Example 1 was applied onto the backside of the mice in an amount of 250 µl per mouse from the thirteenth week. 10% Ethanol containing 1 mM potassium chloride was applied in the same manner to the group applied with the solvent. The administration was once a day, 5 times a week, for 12 consecutive weeks. The observation of changes in wrinkle, and the determinations of the skin elasticity, the thickness of skin and the amount of collagen in skin were carried out in the same manner as in Reference Example 1. The results are shown in Tables 21 to 24. As a result, the group applied with F-fucoidan-containing filtrate significantly ameliorated the formation of wrinkle and also ameliorated the lowering of the skin elasticity, as compared to the group applied with the solvent and the group with UVB irradiation, and further suppressed a decrease in amount of collagen in skin. In addition, an increase in thickness of skin due to UVB was ameliorated. The numerical figures in the tables show an average value ± standard error of 5 cases.

**Table 21**

| Ameliorative Action of Wrinkle Formation on Backside Skin of Mouse by Continuous Application of F-Fucoidan-Containing Filtrate | | |
|---|---|---|
| | Evaluation of Wrinkle | |
| Mouse Group | 12th Week | 24th Week |
| Group Applied with F-Fucoidan-Containing Filtrate (n=5) | 2.15 ± 0.205 | 1.65 ± 0.155 |
| Group Applied with Solvent (n=5) | 3 ± 0.25 | 2.8 ± 0.215 |
| Group with UVB Irradiation (n=5) | 3 ± 0.25 | 2.85 ± 0.225 |
| Normal Control Group (n=5) | 0 | 0 |

**Table 22**

| Action of Improving Skin Elasticity on Backside of Mouse by Continuous Application of F-Fucoidan-Containing Filtrate | | |
|---|---|---|
| Mouse Group Mouse Group | Skin Elasticity | |
| | 12th Week | 24th Week |
| Group Applied with F-Fucoidan-Containing Filtrate (n=5) | 0.641 ± 0.065 | 0.698 ± 0.072 |
| Group Applied with Solvent (n=5) | 0.609 ± 0.065 | 0.571 ± 0.053 |
| Group with UVB Irradiation (n=5) | 0.641 ± 0.065 | 0.567 ± 0.055 |
| Normal Control Group (n=5) | 0.752 ± 0.073 | 0.762 ± 0.067 |
| The value for the theoretical maximum elasticity is 1. | | |

**Table 23**

| Changes in Thickness of Skin on Backside of Mouse by Continuous Application of F-Fucoidan-Containing Filtrate | |
|---|---|
| Mouse Group | Thickness of Skin (µm) 24th Week |
| Group Applied with F-Fucoidan-containing Filtrate (n=5) | 48 ± 3.761 |
| Group Applied with Solvent (n=5) | 96 ± 6.333 |
| Group with UVB Irradiation (n=5) | 106 ± 6.606 |
| Normal Control Group (n=5) | 21 ± 1.909 |
| The values for thickness of skin in the table indicates the distance between stratum corneum epidermis and stratum basale epidermis in the backside. | |

**Table 24**

| Changes in Amount of Collagen in Skin of Mouse by Continuous Application of F-Fucoidan-Containing Filtrate | |
|---|---|
| Mouse Group | Amount of Collagen in Skin (µg/mg) 24th Week |
| Group Applied with F-Fucoidan-Containing Filtrate (n=5) | 39 ± 1.986 |
| Group Applied with Solvent (n=5) | 30 ± 2.712 |
| Group with UVB Irradiation (n=5) | 26 ± 2.513 |
| Normal Control Group (n=5) | 42 ± 1.475 |

### Example 10

(1) Cream-type cosmetics containing the F-fucoidan-containing filtrate described in item (4) of Preparation Example 1 were prepared. Specifically, the F-fucoidan-containing filtrate described in item (4) of Preparation Example 1 was dissolved in a cream (hydrophilic ointment, manufactured by Maruishi Pharmaceutical Co., Ltd.) so as to have a concentration of 25% (v/v), to prepare creamy cosmetics containing F-fucoidan.

(2) Female HOS:HR-1 hairless mice were purchased from Nippon SLC, and used for an experiment from 5 week-old after preliminary rearing. The process and the conditions for generating skin wrinkle-formed models of mice were the same as those for the experimental method described in Reference Example 1. After the termination of the period of wrinkle induction by UVB irradiation, the observation of changes in wrinkle and the determination of the skin elasticity were carried out in the same manner as in Reference Example 1. The creamy cosmetics containing F-fucoidan prepared in item (1) of Example 10 were applied onto the backside of the mice from the thirteenth week in an amount of 250 µl per mouse. Cream alone [distilled water: cream = 25% : 75% (v/v)] was applied in the same manner to the group applied with the solvent. The administration was once per day, 5 times a week, for 12 consecutive weeks. The observation of changes in wrinkle, and the determinations of the skin elasticity, the thickness of skin and the amount of collagen in skin were carried out in the same manner as in Reference Example 1. The results are shown in Tables 25 to 28. "Group Applied with F-Fucoidan Cream" in the tables means the group applied with the creamy cosmetics containing F-fucoidan. The numerical figures in the tables show an average value ± standard error of 5 cases. As a result, the group applied with the creamy cosmetics containing F-fucoidan significantly ameliorated the formation of wrinkle, and improved the loss of the skin elasticity, as compared to those of the group applied with the solvent and the group with UVB irradiation, and further suppressed decrease in amount of collagen in skin. In addition, the group applied with the creamy cosmetics ameliorated an increase in thickness of skin by UVB.

**Table 25**

| Ameliorative Action of Wrinkle on Backside Skin of Mouse by Continuous Application of F-Fucoidan-Containing Creamy Cosmetics | | |
|---|---|---|
| Mouse Group Mouse Group | Evaluation of Wrinkle | |
| | 12th Week | 24th Week |
| Group Applied with F-Fucoidan-Cream (n=5) | 2.75 ± 0.225 | 1.75 ± 0.155 |
| Group Applied with Solvent (n=5) | 2.85 ± 0.205 | 2.55 ± 0.205 |
| Group with UVB Irradiation (n=5) | 3 ± 0.25 | 2.85 ± 0.225 |
| Normal Control Group (n=5) | 0 | 0 |

**Table 26**

| Action of Improving Skin Elasticity of Backside of Mouse by Continuous Application of F-Fucoidan-Containing Creamy Cosmetics | | |
|---|---|---|
| | Skin Elasticity | |
| Mouse Group | 12th Week | 24th Week |
| Group Applied with F-Fucoidan-Cream (n=5) | 0.698 ± 0.075 | 0.771 ± 0.061 |
| Group Applied with Solvent (n=5) | 0.609 ± 0.065 | 0.589 ± 0.054 |
| Group with UVB Irradiation (n=5) | 0.641 ± 0.065 | 0.567 ± 0.055 |
| Normal Control Group (n=5) | 0.752 ± 0.073 | 0.762 ± 0.067 |
| The value for the theoretical maximum elasticity is 1. | | |

**Table 27**

| Changes in Thickness of Skin of Backside of Mouse by Continuous Application of F-Fucoidan-Containing Creamy Cosmetics | |
|---|---|
| | Thickness of Skin (µm) |
| Mouse Group | 24th Week |
| Group Applied with F-Fucoidan-Cream (n=5) | 42 ± 3.965 |
| Group Applied with Solvent (n=5) | 79 ± 6.782 |
| Group with UVB Irradiation (n=5) | 106 ± 6.606 |
| Normal Control Group (n=5) | 21 ± 1.909 |
| The values for thickness of skin in the table indicates the distance between stratum corneum epidermis and stratum basal epidermis in the backside epidermis. | |

**Table 28**

| Changes in Amount of Collagen in Skin of Mouse by Continuous Application of F-Fucoidan-Containing Creamy Cosmetics | |
|---|---|
| Mouse Group | Amount of Collagen in Skin (µg/mg) |
| | 24th Week |
| Group Applied with F-Fucoidan-Cream (n=5) | 47 ± 2.887 |
| Group Applied with Solvent (n=5) | 36 ± 2.116 |
| Group with UVB Irradiation (n=5) | 26 ± 2.513 |
| Normal Control Group (n=5) | 42 ± 1.475 |

### Deposited Biological Materials

(1) Name and Addressee of Depository Authority
   the International Patent Organism Depositary, National Institute of Advanced Industrial Science and Technology
   Tsukuba Central 6, 1-1, Higashi 1-chome Tsukuba-shi, Ibaraki-ken, Japan (Zip code 305-8566)
(2) Deposited Microorganisms
   (i) *Alteromonas* sp. SN-1009
      Original Date of Deposit: February 13, 1996
      Date of Request for Transfer to International Deposit: November 15, 1996
      Accession Number: FERM BP-5747
   (ii) *Flavobacterium* sp. SA-0082
      Original Date of Deposit: March 29, 1995
      Date of Request for Transfer to International Deposit: February 15, 1996
      Accession Number: FERM BP-5402

### INDUSTRIAL APPLICABILITY

According to the present invention, there is provided a medicament for use as any one of a therapeutic agent or prophylactic agent for a disease requiring enhancement of TGF-β production, an agent for amelioration or prevention of wrinkle, an agent for improvement or sustenance of skin elasticity, an agent for amelioration or prevention of epidermal thickening, and a suppressor for a decrease in collagen or an enhancer for collagen production, characterized in that the medicament comprises as an effective ingredient one or more members selected from the group consisting of a fucoidan, a degradation product thereof and a salt thereof.

Furthermore, there are provided cosmetics used for any one of enhancement of TGF-β production, amelioration or prevention of wrinkle, improvement or sustenance of skin elasticity, amelioration or prevention of epidermal thickening, and suppression for decreasing collagen or enhancement for collagen production, characterized in that the cosmetics comprise as an effective ingredient one or more members selected from the group consisting of a fucoidan, a degradation product thereof and a salt thereof.

Also, there is provided any one of a method for enhancement of TGF-β production, a method for amelioration or prevention of wrinkle, a method for improvement or sustenance of skin elasticity, a method for amelioration or prevention of epidermal thickening, and a method for suppression for decreasing collagen or enhancement for collagen production, characterized by administering to an individual one or more members selected from the group consisting of a fucoidan, a degradation product thereof and a salt thereof.

Moreover, there is provided use of one or more members selected from the group consisting of a fucoidan, a degradation product thereof and a salt thereof, for manufacturing a medicament for any one of enhancement of TGF-β production, amelioration or prevention of wrinkle, improvement or sustenance of skin elasticity, amelioration or prevention of epidermal thickening, and suppression for decreasing collagen or enhancement for collagen production.

Therefore, according to the present invention, there can be achieved treatment or prevention of a disease requiring enhancement of TGF-β production, amelioration or prevention of wrinkle, improvement or sustenance of skin elasticity, amelioration or prevention of epidermal thickening, and/or suppression for decreasing collagen or enhancement for collagen production in the skin.

## Claims

1. A medicament for use as any one of a therapeutic agent or prophylactic agent for a disease requiring enhancement of transforming growth factor-β production, an agent for amelioration or prevention of wrinkle, an agent for improvement or sustenance of skin elasticity, an agent for amelioration or prevention of epidermal thickening, and a suppressor for decreasing collagen or enhancer for collagen production, **characterized in that** the medicament comprises as an effective ingredient one or more members selected from the group consisting of a fucoidan, a degradation product thereof and a salt thereof.

2. The medicament according to claim 1, wherein the fucoidan is derived from algae or from Echinodermata.

3. The medicament according to claim 2, wherein the algae are Phaeophycae.

4. Cosmetics used for any one of enhancement of transforming growth factor-β production, amelioration or prevention of wrinkle, improvement or sustenance of skin elasticity, amelioration or prevention of epidermal thickening, and suppression for decreasing collagen or enhancement for collagen production, **characterized in that** the cosmetics comprise as an effective ingredient one or more members selected from the group consisting of a fucoidan, a degradation product thereof and a salt thereof.

5. The cosmetics according to claim 4, wherein the fucoidan is derived from algae or from Echinodermata.

6. The cosmetics according to claim 5, wherein the algae are Phaeophycae.

7. The cosmetics according to any one of claims 4 to 6, wherein the cosmetics are in the form of a lotion, a milky lotion, cream, a facial pack, a bathing agent, a facial cleansing agent, a bath detergent, a bathing soap or an ointment.

8. A method selected from a method for enhancement of transforming growth factor-β production, a method for amelioration or prevention of wrinkle, a method for improvement or sustenance of skin elasticity, a method for amelioration or prevention of epidermal thickening, and a method for suppression for decreasing collagen or enhancement of collagen production, **characterized by** administering to an individual one or more members selected from the group consisting of a fucoidan, a degradation product thereof and a salt thereof.

9. The method according to claim 8, wherein the fucoidan is derived from algae or from Echinodermata.

10. The method according to claim 9, wherein the algae are Phaeophycae.

11. Use of one or more members selected from the group consisting of a fucoidan, a degradation product thereof and a salt thereof, for manufacturing a medicament for any one of enhancement of transforming growth factor-β production, amelioration or prevention of wrinkle, improvement or sustenance of skin elasticity, amelioration or prevention of epidermal thickening, and suppression for decreasing collagen or enhancement of collagen production.
